# EUROPEAN PATENT APPLICATION

(11) **EP 2 210 944 A1**
(43) Date of publication of application: **28.07.2010**
(21) Application number: 09000893.9
(22) Date of filing: 22.01.2009
(51) Int. Cl.: C12N 15/10

(54) **Methods for generation of RNA and (poly)peptide libraries and their use**

(71) Applicant: ATG:biosynthetics GmbH, 79249 Merzhausen (DE)
(72) Inventor: Lebens, Michael, 52399 Hökerum (SE)
(74) Representative: Vossius & Partner

(57) **Abstract**

The present invention relates to a method for generating an RNA library or a (poly)peptide library comprising the steps of: (a) providing one or more nucleic acid molecules each comprising i) two or more coding elements (A) each giving rise to an RNA molecule upon transcription and/or a (poly)peptide upon transcription and translation; and ii) linking elements (B) arranged according to the general formula of B(AB)₂₊ₙ, wherein said linking elements comprise one or more sequence motifs not found in said two or more coding elements allowing specific disruption of the linking elements (B); (b) cloning the nucleic acid molecule of step (a) into a vector; (c) transforming a host cell with the vector obtained in step (b) and propagating said transformed cell; (d) preparing vector DNA from the transformed and propagated cells of step (c); (e) (i) disrupting the vector DNA obtained in step (d) with one or more agents recognizing said one or more sequence motifs of the linking elements or (ii) performing an amplification step with the vector DNA obtained in step (d) and primers hybridizing to the sequence of said linking elements so that the sequences comprising the coding elements (A) are specifically amplified; (f) cloning the resulting coding elements (A) of step (e) into vectors; (g) transforming the vectors obtained in step (f) into host cells and establishing clonal colonies; and (h) culturing said clonal colonies under conditions suitable to express the coding elements. Also, the method relates to an RNA library or a (poly)peptide library obtainable or obtained according to the method of the invention. Moreover, the invention relates to a method for identifying a (poly)peptide epitope recognized by an antibody or a (poly)peptide-binding compound and a method for identifying a (poly)peptide epitope recognized by antibodies in serum. Further, the invention relates to a method for generating protein variants. Finally, the invention relates to a nucleic acid molecule a used in the method of the invention, a vector comprising the same, a cell comprising said nucleic acid molecule or said vector and a kit comprising one or more items selected from the group of said nucleic acid molecule, said vector, said cell, said RNA or a (poly)peptide library of the invention and, optionally, instructions for use.

SEQUENCE LISTING

<110>  ATG:biosynthetics GmbH

<120>  Methods for generation of RNA and (poly)peptide libraries and
       their use

<130>  R1042 EP

<160>  108

<170>  PatentIn version 3.3

<210>  1
<211>  98
<212>  PRT
<213>  human papilloma virus

<400>  1

Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp
        35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

Lys Pro

<210>  2
<211>  96
<212>  PRT
<213>  artificial sequence

<220>
<223>  /note="Description of artificial sequence: HPV16 overlapping
       peptide sequence"

<400>  2

Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp

35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

<210>  3
<211>  96
<212>  PRT
<213>  artificial sequence

<220>
<223>  /note="Description of artificial sequence: HPV16 overlapping
       peptide sequence"

<400>  3

Thr Pro Thr Leu His Glu Tyr Met Leu Asp Leu Gln Pro Glu Thr Thr
1               5                   10                  15

Asp Leu Tyr Cys Tyr Glu Gln Leu Asn Asp Ser Ser Glu Glu Glu Asp
            20                  25                  30

Glu Ile Asp Gly Pro Ala Gly Gln Ala Glu Pro Asp Arg Ala His Tyr
        35                  40                  45

Asn Ile Val Thr Phe Cys Cys Lys Cys Asp Ser Thr Leu Arg Leu Cys
    50                  55                  60

Val Gln Ser Thr His Val Asp Ile Arg Thr Leu Glu Asp Leu Leu Met
65                  70                  75                  80

Gly Thr Leu Gly Leu Gly Ile Val Cys Pro Ile Cys Ser Gln Lys Pro
                85                  90                  95

<210>  4
<211>  84
<212>  PRT
<213>  artificial sequence

<220>
<223>  /note="Description of artificial sequence: HPV16 overlapping
       peptide sequence"

<400>  4

His Glu Tyr Met Leu Asp Leu Gln Pro Glu Thr Thr Asp Leu Tyr Cys
1               5                   10                  15

Tyr Glu Gln Leu Asn Asp Ser Ser Glu Glu Glu Asp Glu Ile Asp Gly
            20                  25                  30

Pro Ala Gly Gln Ala Glu Pro Asp Arg Ala His Tyr Asn Ile Val Thr
        35                  40                  45

Phe Cys Cys Lys Cys Asp Ser Thr Leu Arg Leu Cys Val Gln Ser Thr
    50                  55                  60

His Val Asp Ile Arg Thr Leu Glu Asp Leu Leu Met Gly Thr Leu Gly
65                  70                  75                  80

Ile Val Cys Pro

<210>  5
<211>  382
<212>  DNA
<213>  artificial sequence

<220>
<223>  /note="Description of artificial sequence: synthesized HPV16
       nucleic acid molecule"

<400>  5
aagctttgcc atgcatggag atacacctac attgcatgaa tatatgcctg caggtggttt      60

ctgagaacag atggggcaca caattcctag ggcaaagctt tgccttagat ttgcaaccag     120

agacaactga tctctactgt cctgcaggtc ctagtgtgcc cattaacagg tcttccaaag     180

tacgggcaaa gctttgccta tgagcaatta aatgacagct cagaggagga ggatcctgca     240

ggcgtagagt cacacttgca acaaaaggtt acaatattgg caaagctttg ccgaaataga     300

tggtccagct ggacaagcag aaccggaccc tgcaggaatg tctacgtgtg tgctttgtac     360

gcacaaccga agggcaaagc tt                                              382

<210>  6
<211>  382
<212>  DNA
<213>  artificial sequence

<220>
<223>  /note="Description of artificial sequence: synthesized HPV16
       nucleic acid molecule"

<400>  6
aagctttgcc agagcccatt acaatattgt aaccttttgt tgcaagcctg caggaccatc      60

tatttcatcc tcctcctctg agctgtcatt ggcaaagctt tgcctgtgac tctacgcttc     120

ggttgtgcgt acaaagcaca cctgcagggt aatgggctct gtccggttct gcttgtccag     180

ctggggcaaa gctttgccca cgtagacatt cgtactttgg aagacctgtt aatgcctgca     240

ggtaattgct cataacagta gagatcagtt gtctctgggg caaagctttg ccggcacact     300

aggaattgtg tgccccatct gttctcagcc tgcaggttgc aaatctaaca tatattcatg     360

caatgtaggt gtggcaaagc tt                                              382

<210>  7

<211>  335
<212>  DNA
<213>  artificial sequence

<220>
<223>  /note="Description of artificial sequence: synthesized HPV16
       nucleic acid molecule"

<400>  7
aagctttgcc catgaatata tgttagattt gcaaccagag acaactcctg caggggggca      60

cacaattcct agtgtgccca ttaacaggtc ggcaaagctt tgccgatctc tactgttatg     120

agcaattaaa tgacagctca cctgcaggtt ccaaagtacg aatgtctacg tgtgtgcttt     180

gtacggcaaa gctttgccga ggaggaggat gaaatagatg gtccagctgg acaacctgca     240

gggcacaacc gaagcgtaga gtcacacttg caacaaaagg caaagctttg ccgcagaacc     300

ggacagagcc cattacaata ttgtaacccc tgcag                                335


<210>  8
<211>  12
<212>  PRT
<213>  artificial sequence

<220>
<223>  /note="Description of artificial sequence: HPV antibody binding
       sequence"

<400>  8

Glu Ile Asp Gly Pro Ala Gly Gln Ala Glu Pro Asp
1               5                   10


<210>  9
<211>  12
<212>  PRT
<213>  artificial sequence

<220>
<223>  /note="Description of artificial sequence: HPV antibody binding
       sequence"

<400>  9

Met His Gly Asp Thr Pro Thr Leu His Glu Tyr Met
1               5                   10


<210>  10
<211>  12
<212>  PRT
<213>  artificial sequence

<220>
<223>  /note="Description of artificial sequence: HPV antibody binding
       sequence"

<400>  10

Thr Pro Thr Leu His Glu Tyr Met Leu Asp Leu Gln
1               5                   10


<210>  11

<211>  8
<212>  PRT
<213>  artificial sequence

<220>
<223>  /note="Description of artificial sequence: HPV antibody binding
       sequence"

<400>  11

Thr Pro Thr Leu His Glu Tyr Met
1               5


<210>  12
<211>  98
<212>  PRT
<213>  human papilloma virus

<400>  12

Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp
        35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

Lys Pro


<210>  13
<211>  105
<212>  PRT
<213>  human papilloma virus

<400>  13

Met His Gly Pro Lys Ala Thr Leu Gln Asp Ile Val Leu His Leu Glu
1               5                   10                  15

Pro Gln Asn Glu Ile Pro Val Asp Leu Leu Cys His Glu Gln Leu Ser
            20                  25                  30

Asp Ser Glu Glu Glu Asn Asp Glu Ile Asp Gly Val Asn His Gln His
        35                  40                  45

Leu Pro Ala Arg Arg Ala Glu Pro Gln Arg His Thr Met Leu Cys Met
    50                  55                  60

Cys Cys Lys Cys Glu Ala Arg Ile Glu Leu Val Val Glu Ser Ser Ala
65                  70                  75                  80

Asp Asp Leu Arg Ala Phe Gln Gln Leu Phe Leu Asn Thr Leu Ser Phe
                85                  90                  95

Val Cys Pro Trp Cys Ala Ser Gln Gln
            100                 105

<210>  14
<211>  294
<212>  DNA
<213>  human papilloma virus

<400>  14
atgcatggag atacacctac attgcatgaa tatatgttag atttgcaacc agagacaact      60

gatctctact gttatgagca attaaatgac agctcagagg aggaggatga aatagatggt     120

ccagctggac aagcagaacc ggacagagcc cattacaata ttgtaacctt ttgttgcaag     180

tgtgactcta cgcttcggtt gtgcgtacaa agcacacacg tagacattcg tactttggaa     240

gacctgttaa tgggcacact aggaattgtg tgccccatct gttctcagaa acca           294

<210>  15
<211>  12
<212>  PRT
<213>  artificial sequence

<220>
<223>  /note="Description of artificial sequence: HPV antibody binding
       sequence"

<400>  15

Met His Gly Asp Thr Pro Thr Leu His Glu Tyr Met
1               5                   10

<210>  16
<211>  12
<212>  PRT
<213>  artificial sequence

<220>
<223>  /note="Description of artificial sequence: HPV antibody binding
       sequence"

<400>  16

Thr Pro Thr Leu His Glu Tyr Met Leu Asp Leu Gln
1               5                   10

<210>  17
<211>  12
<212>  PRT
<213>  artificial sequence

<220>
<223>   /note="Description of artificial sequence: HPV antibody binding
        sequence"

<400>   17

Leu Asp Leu Gln Pro Glu Thr Thr Asp Leu Tyr Cys
1               5                   10


<210>   18
<211>   12
<212>   PRT
<213>   artificial sequence

<220>
<223>   /note="Description of artificial sequence: HPV antibody binding
        sequence"

<400>   18

Glu Ile Asp Gly Pro Ala Gly Gln Ala Glu Pro Asp
1               5                   10


<210>   19
<211>   12
<212>   PRT
<213>   artificial sequence

<220>
<223>   /note="Description of artificial sequence: HPV antibody binding
        sequence"

<400>   19

Arg Ala His Tyr Asn Ile Val Thr Phe Cys Cys Lys
1               5                   10


<210>   20
<211>   12
<212>   PRT
<213>   artificial sequence

<220>
<223>   /note="Description of artificial sequence: HPV antibody binding
        sequence"

<400>   20

Phe Cys Cys Lys Cys Asp Ser Thr Leu Arg Leu Cys
1               5                   10


<210>   21
<211>   12
<212>   PRT
<213>   artificial sequence

<220>
<223>   /note="Description of artificial sequence: HPV antibody binding
        sequence"

<400>   21

Val Gln Ser Thr His Val Asp Ile Arg Thr Leu Glu
1               5                   10

<210> 22
<211> 49
<212> DNA
<213> artificial sequence

<220>
<223> /note="Description of artificial sequence: insert sequence"

<400> 22
aagctttgcc catgaatata tgttagattt gcaaccagag acaactcct 49


<210> 23
<211> 49
<212> DNA
<213> artificial sequence

<220>
<223> /note="Description of artificial sequence: insert sequence"

<400> 23
aagctttgcc catgaatata tgttagattt gcaaccagag acaactcct 49


<210> 24
<211> 49
<212> DNA
<213> artificial sequence

<220>
<223> /note="Description of artificial sequence: insert sequence"

<400> 24
aagctttgcc gcagaaccgg acagagccca ttacaatatt gtaacccct 49


<210> 25
<211> 49
<212> DNA
<213> artificial sequence

<220>
<223> /note="Description of artificial sequence: insert sequence"

<400> 25
aagctttgcc catgaatata tgttagattt gcaaccagag acaactcct 49


<210> 26
<211> 49
<212> DNA
<213> artificial sequence

<220>
<223> /note="Description of artificial sequence: insert sequence"

<400> 26
aagctttgcc atgcatggag atacacctac attgcatgaa tatatgcct 49


<210> 27
<211> 49
<212> DNA
<213> artificial sequence

<220>

<223>  /note="Description of artificial sequence: insert sequence"

<400>  27
aagctttgcc catgaatata tgttagattt gcaaccagag acaactcct                49


<210>  28
<211>  49
<212>  DNA
<213>  artificial sequence

<220>
<223>  /note="Description of artificial sequence: insert sequence"

<400>  28
aagctttgcc catgaatata tgttagattt gcaaccagag acaactcct                49


<210>  29
<211>  49
<212>  DNA
<213>  artificial sequence

<220>
<223>  /note="Description of artificial sequence: insert sequence"

<400>  29
aagctttgcc acacctacat tgcatgaata tatgttagat ttgcaacct                49


<210>  30
<211>  49
<212>  DNA
<213>  artificial sequence

<220>
<223>  /note="Description of artificial sequence: insert sequence"

<400>  30
aagctttgcc catgaatata tgttagattt gcaaccagag acaactcct                49


<210>  31
<211>  49
<212>  DNA
<213>  artificial sequence

<220>
<223>  /note="Description of artificial sequence: insert sequence"

<400>  31
aagctttgcc cacgtagaca ttcgtacttt ggaagacctg ttaatgcct                49


<210>  32
<211>  49
<212>  DNA
<213>  artificial sequence

<220>
<223>  /note="Description of artificial sequence: insert sequence"

<400>  32
aagctttgcc ttagatttgc aaccagagac aactgatctc tactgtcct                49


<210>  33
<211>  49

<212>  DNA
<213>  artificial sequence

<220>
<223>  /note="Description of artificial sequence: insert sequence"

<400>  33
aagctttgcc ggcacactag gaattgtgtg ccccatctgt tctcagcct                49


<210>  34
<211>  49
<212>  DNA
<213>  artificial sequence

<220>
<223>  /note="Description of artificial sequence: insert sequence"

<400>  34
   aagctttgcc gaaatagatg gtccagctgg acaagcagaa ccggaccct                49


<210>  35
<211>  49
<212>  DNA
<213>  artificial sequence

<220>
<223>  /note="Description of artificial sequence: insert sequence"

<400>  35
aagctttgcc catgaatata tgttagattt gcaaccagag acaactcct                49


<210>  36
<211>  49
<212>  DNA
<213>  artificial sequence

<220>
<223>  /note="Description of artificial sequence: insert sequence"

<400>  36
aagctttgcc tgtgactcta cgcttcggtt gtgcgtacaa agcacacct                49


<210>  37
<211>  12
<212>  PRT
<213>  artificial sequence

<220>
<223>  /note="Description of artificial sequence: HPV antibody binding
       sequence"

<400>  37

His Glu Tyr Met Leu Asp Leu Gln Pro Glu Thr Thr
1               5                   10


<210>  38
<211>  12
<212>  PRT
<213>  artificial sequence

<220>
<223>  /note="Description of artificial sequence: HPV antibody binding

sequence"

<400> 38

His Glu Tyr Met Leu Asp Leu Gln Pro Glu Thr Thr
1               5                   10


<210> 39
<211> 12
<212> PRT
<213> artificial sequence

<220>
<223> /note="Description of artificial sequence: HPV antibody binding
sequence"

<400> 39

Ala Glu Pro Asp Arg Ala His Tyr Asn Ile Val Thr
1               5                   10


<210> 40
<211> 12
<212> PRT
<213> artificial sequence

<220>
<223> /note="Description of artificial sequence: HPV antibody binding
sequence"

<400> 40

His Glu Tyr Met Leu Asp Leu Gln Pro Glu Thr Thr
1               5                   10


<210> 41
<211> 12
<212> PRT
<213> artificial sequence

<220>
<223> /note="Description of artificial sequence: HPV antibody binding
sequence"

<400> 41

Met His Gly Asp Thr Pro Thr Leu His Glu Tyr Met
1               5                   10


<210> 42
<211> 12
<212> PRT
<213> artificial sequence

<220>
<223> /note="Description of artificial sequence: HPV antibody binding
sequence"

<400> 42

His Glu Tyr Met Leu Asp Leu Gln Pro Glu Thr Thr
1               5                   10

<210>  43
<211>  12
<212>  PRT
<213>  artificial sequence

<220>
<223>  /note="Description of artificial sequence: HPV antibody binding
       sequence"

<400>  43

His Glu Tyr Met Leu Asp Leu Gln Pro Glu Thr Thr
1               5                   10


<210>  44
<211>  12
<212>  PRT
<213>  artificial sequence

<220>
<223>  /note="Description of artificial sequence: HPV antibody binding
       sequence"

<400>  44

Thr Pro Thr Leu His Glu Tyr Met Leu Asp Leu Gln
1               5                   10


<210>  45
<211>  12
<212>  PRT
<213>  artificial sequence

<220>
<223>  /note="Description of artificial sequence: HPV antibody binding
       sequence"

<400>  45

His Glu Tyr Met Leu Asp Leu Gln Pro Glu Thr Thr
1               5                   10


<210>  46
<211>  12
<212>  PRT
<213>  artificial sequence

<220>
<223>  /note="Description of artificial sequence: HPV antibody binding
       sequence"

<400>  46

His Val Asp Ile Arg Thr Leu Glu Asp Leu Leu Met
1               5                   10


<210>  47
<211>  12
<212>  PRT
<213>  artificial sequence

<220>
<223>  /note="Description of artificial sequence: HPV antibody binding
       sequence"

<400>  47

Leu Asp Leu Gln Pro Glu Thr Thr Asp Leu Tyr Cys
1               5                   10


<210>  48
<211>  12
<212>  PRT
<213>  artificial sequence

<220>
<223>  /note="Description of artificial sequence: HPV antibody binding
       sequence"

<400>  48

Gly Thr Leu Gly Ile Val Cys Pro Ile Cys Ser Gln
1               5                   10


<210>  49
<211>  12
<212>  PRT
<213>  artificial sequence

<220>
<223>  /note="Description of artificial sequence: HPV antibody binding
       sequence"

<400>  49

Glu Ile Asp Gly Pro Ala Gly Gln Ala Glu Pro Asp
1               5                   10


<210>  50
<211>  12
<212>  PRT
<213>  artificial sequence

<220>
<223>  /note="Description of artificial sequence: HPV antibody binding
       sequence"

<400>  50

His Glu Tyr Met Leu Asp Leu Gln Pro Glu Thr Thr
1               5                   10


<210>  51
<211>  12
<212>  PRT
<213>  artificial sequence

<220>
<223>  /note="Description of artificial sequence: HPV antibody binding
       sequence"

<400>  51

Cys Asp Ser Thr Leu Arg Leu Cys Val Gln Ser Thr
1               5                   10


<210>  52

<211>  12
<212>  PRT
<213>  artificial sequence

<220>
<223>  /note="Description of artificial sequence: HPV antibody binding
       sequence"

<400>  52

His Glu Tyr Met Leu Asp Leu Gln Pro Glu Thr Thr
1               5                   10


<210>  53
<211>  12
<212>  PRT
<213>  artificial sequence

<220>
<223>  /note="Description of artificial sequence: HPV antibody binding
       sequence"

<400>  53

Ala Glu Pro Asp Arg Ala His Tyr Asn Ile Val Thr
1               5                   10


<210>  54
<211>  12
<212>  PRT
<213>  artificial sequence

<220>
<223>  /note="Description of artificial sequence: HPV antibody binding
       sequence"

<400>  54

His Val Asp Ile Arg Thr Leu Glu Asp Leu Leu Met
1               5                   10


<210>  55
<211>  12
<212>  PRT
<213>  artificial sequence

<220>
<223>  /note="Description of artificial sequence: HPV antibody binding
       sequence"

<400>  55

His Glu Tyr Met Leu Asp Leu Gln Pro Glu Thr Thr
1               5                   10


<210>  56
<211>  12
<212>  PRT
<213>  artificial sequence

<220>
<223>  /note="Description of artificial sequence: HPV antibody binding
       sequence"

<400>  56

Leu Asp Leu Gln Pro Glu Thr Thr Asp Leu Tyr Cys
1               5                   10


<210>  57
<211>  12
<212>  PRT
<213>  artificial sequence

<220>
<223>  /note="Description of artificial sequence: HPV antibody binding
       sequence"

<400>  57

Cys Asp Ser Thr Leu Arg Leu Cys Val Gln Ser Thr
1               5                   10


<210>  58
<211>  12
<212>  PRT
<213>  artificial sequence

<220>
<223>  /note="Description of artificial sequence: HPV antibody binding
       sequence"

<400>  58

Gly Thr Leu Gly Ile Val Cys Pro Ile Cys Ser Gln
1               5                   10


<210>  59
<211>  60
<212>  DNA
<213>  artificial sequence

<220>
<223>  /note="Description of artificial sequence: HPV LTB partial
       sequence"

<400>  59
atgaataaag taaaatttta tgttttattt acggcgttac tatcctctct atgtgcacac      60


<210>  60
<211>  327
<212>  DNA
<213>  Mycobacterium tuberculosis

<400>  60
ggagctcctc aaaatattac tgatttgtgt gcagaatacc acaacacaca aatacatacg      60

ctaaatgata agatattttc gtatacagaa tctttggcag gaaaaagaga gatggctatc     120

attactttta agaatggtgc aacttttcaa gtagaagtac caggtagtca acatatagat     180

tcacaaaaaa aagcgataga aaggatgaag gataccctga ggattgcata tcttactgaa     240

gctaaagtcg aaaagttatg tgtatggaat aataaaacgc ctcatgcgat tgccgcaatt     300

agtatggcaa actaactagt taagctt                                         327

<210>  61
<211>  104
<212>  PRT
<213>  Mycobacterium tuberculosis

<400>  61

Gly Ala Pro Gln Ser Ile Thr Glu Leu Cys Ser Glu Tyr His Asn Thr
1               5                   10                  15

Gln Ile Tyr Thr Ile Asn Asp Lys Ile Leu Ser Tyr Thr Glu Ser Met
            20                  25                  30

Ala Gly Lys Arg Glu Met Val Ile Ile Thr Phe Lys Ser Gly Ala Thr
        35                  40                  45

Phe Gln Val Glu Val Pro Gly Ser Gln His Ile Asp Ser Gln Lys Lys
    50                  55                  60

Ala Ile Glu Arg Met Lys Asp Thr Leu Arg Ile Thr Tyr Leu Thr Glu
65                  70                  75                  80

Thr Lys Ile Asp Lys Leu Cys Val Trp Asn Asn Lys Thr Pro Asn Ser
                85                  90                  95

Ile Ala Ala Ile Ser Met Glu Asn
            100

<210>  62
<211>  104
<212>  PRT
<213>  Mycobacterium tuberculosis

<400>  62

Gly Ala Pro Gln Asn Ile Thr Asp Leu Cys Ala Glu Tyr His Asn Thr
1               5                   10                  15

Gln Ile His Thr Leu Asn Asp Lys Ile Phe Ser Tyr Thr Glu Ser Leu
            20                  25                  30

Ala Gly Lys Arg Glu Met Ala Ile Ile Thr Phe Lys Asn Gly Ala Thr
        35                  40                  45

Phe Gln Val Glu Val Pro Gly Ser Gln His Ile Asp Ser Gln Lys Lys
    50                  55                  60

Ala Ile Glu Arg Met Lys Asp Thr Leu Arg Ile Ala Tyr Leu Thr Glu
65                  70                  75                  80

Ala Lys Val Glu Lys Leu Cys Val Trp Asn Asn Lys Thr Pro His Ala
                85                  90                  95

Ile Ala Ala Ile Ser Met Ala Asn
            100

<210>  63
<211>  104
<212>  PRT
<213>  Mycobacterium tuberculosis

<400>  63

Gly Ala Pro Gln Asn Ile Thr Glu Leu Cys Ser Glu Tyr His Asn Thr
1               5                   10                  15

Gln Ile Tyr Glu Leu Asn Lys Glu Ile Lys Thr Tyr Thr Glu Ser Leu
            20                  25                  30

Ala Gly Tyr Arg Glu Met Val Ile Ile Ser Phe Ala Asn Gly Ala Thr
        35                  40                  45

Phe Gln Val Glu Val Pro Gly Ser Gln His Leu Glu Ser Gln Lys Arg
    50                  55                  60

Pro Leu Glu Arg Met Lys Asp Thr Leu Arg Ala Ala Tyr Phe Thr Gly
65                  70                  75                  80

Ile Lys Val Ser Lys Leu Cys Val Trp Asn Asn Lys Thr Pro Asn Ser
                85                  90                  95

Ile Ala Ala Ile Glu Leu Ser Asn
            100

<210>  64
<211>  41
<212>  DNA
<213>  artificial sequence

<220>
<223>  /note="Description of artificial sequence: toxin B subunit
       constant region"

<400>  64
ggcgttacta tcctctctat gtgcacacgg agctcctcaa a                          41

<210>  65
<211>  23
<212>  DNA
<213>  artificial sequence

<220>
<223>  /note="Description of artificial sequence: toxin B subunit
       constant region"

<400>  65
cagaatacca caacacacaa ata                                              23

<210>  66
<211>  39
<212>  DNA
<213>  artificial sequence

<220>
<223>   /note="Description of artificial sequence: toxin B subunit
        constant region"

<400>   66
tggtgcaact tttcaagtag aagtaccagg tagtcaaca                              39


<210>   67
<211>   26
<212>   DNA
<213>   artificial sequence

<220>
<223>   /note="Description of artificial sequence: toxin B subunit
        constant region"

<400>   67
tagaaaggat gaaggatacc ctgagg                                            26


<210>   68
<211>   29
<212>   DNA
<213>   artificial sequence

<220>
<223>   /note="Description of artificial sequence: toxin B subunit
        constant region"

<400>   68
aagttatgtg tatggaataa taaaacgcc                                         29


<210>   69
<211>   177
<212>   DNA
<213>   artificial sequence

<220>
<223>   /note="Description of artificial sequence: synthetic nucleic acid
        molecule carrying all toxin B constant regions"

<400>   69
ggcgccagaa taccacaaca cacaaatagc gccgttacta tcctctctat gtgcacacgg      60

agctcctcaa agcgctggtg caacttttca agtagaagta ccaggtagtc aacagcgcta     120

gaaaggatga aggataccct gagggcgctt atgtgtatgg aataataaaa cgccggc       177


<210>   70
<211>   55
<212>   DNA
<213>   artificial sequence

<220>
<223>   /note="Description of artificial sequence: toxin B subunit
        variant region"

<400>   70
ctgcaggagc tcctcaaaat attactgatt tgtgtgcaga ataccacaac tgcag            55


<210>   71
<211>   66
<212>   DNA
<213>   artificial sequence

<220>
<223>  /note="Description of artificial sequence: toxin B subunit
       variant region"

<400>  71
atgcatacca caacacacaa atacatacgc taaatgataa gatattttcg tatacagaat      60

ctgcag                                                                  66


<210>  72
<211>  66
<212>  DNA
<213>  artificial sequence

<220>
<223>  /note="Description of artificial sequence: toxin B subunit
       variant region"

<400>  72
atgcatacca caacacacaa atacatgaac taaatgataa gatattttcg tatacagaat      60

ctgcag                                                                  66


<210>  73
<211>  66
<212>  DNA
<213>  artificial sequence

<220>
<223>  /note="Description of artificial sequence: toxin B subunit
       variant region"

<400>  73
atgcatacca caacacacaa atacatacgc taaataagaa gatattttcg tatacagaat      60

ctgcag                                                                  66


<210>  74
<211>  66
<212>  DNA
<213>  artificial sequence

<220>
<223>  /note="Description of artificial sequence: toxin B subunit
       variant region"

<400>  74
atgcatacca caacacacaa atacatgaac taaataagaa gatattttcg tatacagaat      60

ctgcag                                                                  66


<210>  75
<211>  66
<212>  DNA
<213>  artificial sequence

<220>
<223>  /note="Description of artificial sequence: toxin B subunit
       variant region"

<400>  75
atgcatacca caacacacaa atacatacgc taaatgataa gataaagtcg tatacagaat      60

ctgcag                                                                66

<210>  76
<211>  66
<212>  DNA
<213>  artificial sequence

<220>
<223>  /note="Description of artificial sequence: toxin B subunit
       variant region"

<400>  76
atgcatacca caacacacaa atacatgaac taaatgataa gataaagtcg tatacagaat      60

ctgcag                                                                66

<210>  77
<211>  66
<212>  DNA
<213>  artificial sequence

<220>
<223>  /note="Description of artificial sequence: toxin B subunit
       variant region"

<400>  77
atgcatacca caacacacaa atacatacgc taaataagaa gataaagtcg tatacagaat      60

ctgcag                                                                66

<210>  78
<211>  66
<212>  DNA
<213>  artificial sequence

<220>
<223>  /note="Description of artificial sequence: toxin B subunit
       variant region"

<400>  78
atgcatacca caacacacaa atacatgaac taaataagaa gataaagtcg tatacagaat      60

ctgcag                                                                66

<210>  79
<211>  78
<212>  DNA
<213>  artificial sequence

<220>
<223>  /note="Description of artificial sequence: toxin B subunit
       variant region"

<400>  79
ctgcagtata cagaatcttt ggcaggaaaa agagagatgg ctatcattac ttttaagaat      60

ggtgcaactt ttctgcag                                                   78

<210>  80
<211>  78
<212>  DNA
<213>  artificial sequence

<220>
<223>  /note="Description of artificial sequence: toxin B subunit
      variant region"

<400>  80
ctgcagtata cagaatcttt ggcaggatac agagagatgg ctatcattac ttttaagaat      60

ggtgcaactt ttctgcag                                                    78


<210>  81
<211>  78
<212>  DNA
<213>  artificial sequence

<220>
<223>  /note="Description of artificial sequence: toxin B subunit
      variant region"

<400>  81
ctgcagtata cagaatcttt ggcaggaaaa agagagatgg ctatcattac ttttgcgaat      60

ggtgcaactt ttctgcag                                                    78


<210>  82
<211>  78
<212>  DNA
<213>  artificial sequence

<220>
<223>  /note="Description of artificial sequence: toxin B subunit
      variant region"

<400>  82
ctgcagtata cagaatcttt ggcaggatac agagagatgg ctatcattac ttttgcgaat      60

ggtgcaactt ttctgcag                                                    78


<210>  83
<211>  65
<212>  DNA
<213>  artificial sequence

<220>
<223>  /note="Description of artificial sequence: toxin B subunit
      variant region"

<400>  83
ctgcagtacc aggtagtcaa catatagatt cacaaaaaaa agcgatagaa aggatgaagg      60

atgca                                                                  65


<210>  84
<211>  68
<212>  DNA
<213>  artificial sequence

<220>
<223>  /note="Description of artificial sequence: toxin B subunit
      variant region"

<400>  84
ctgcagatac cctgaggatt gcatatctta ctgaagctaa agtcgaaaag ttatgtgtat      60

ggatgcat                                                               68

<210>  85
<211>  68
<212>  DNA
<213>  artificial sequence

<220>
<223>  /note="Description of artificial sequence: toxin B subunit
       variant region"

<400>  85
ctgcagatac cctgagggct gcatatctta ctgaagctaa agtcgaaaag ttatgtgtat      60

ggatgcat                                                                68


<210>  86
<211>  67
<212>  DNA
<213>  artificial sequence

<220>
<223>  /note="Description of artificial sequence: toxin B subunit
       variant region"

<400>  86
ctgcagatac cctgaggatt gcatatctta ctgaaattaa agtcgaaaag ttatgtgtat      60

ggtgcat                                                                 67


<210>  87
<211>  68
<212>  DNA
<213>  artificial sequence

<220>
<223>  /note="Description of artificial sequence: toxin B subunit
       variant region"

<400>  87
ctgcagatac cctgagggct gcatatctta ctgaaattaa agtcgaaaag ttatgtgtat      60

ggatgcat                                                                68


<210>  88
<211>  68
<212>  DNA
<213>  artificial sequence

<220>
<223>  /note="Description of artificial sequence: toxin B subunit
       variant region"

<400>  88
ctgcagatac cctgaggatt gcatatctta ctgaagctaa agtctctaag ttatgtgtat      60

ggatgcat                                                                68


<210>  89
<211>  68
<212>  DNA
<213>  artificial sequence

<220>

<223>  /note="Description of artificial sequence: toxin B subunit
      variant region"

<400>  89
ctgcagatac cctgagggct gcatatctta ctgaagctaa agtctctaag ttatgtgtat    60

ggatgcat                                                              68


<210>  90
<211>  68
<212>  DNA
<213>  artificial sequence

<220>
<223>  /note="Description of artificial sequence: toxin B subunit
      variant region"

<400>  90
ctgcagatac cctgaggatt gcatatctta ctgaaattaa agtctctaag ttatgtgtat    60

ggatgcat                                                              68


<210>  91
<211>  68
<212>  DNA
<213>  artificial sequence

<220>
<223>  /note="Description of artificial sequence: toxin B subunit
      variant region"

<400>  91
ctgcagatac cctgagggct gcatatctta ctgaaattaa agtctctaag ttatgtgtat    60

ggatgcat                                                              68


<210>  92
<211>  80
<212>  DNA
<213>  artificial sequence

<220>
<223>  /note="Description of artificial sequence: toxin B subunit
      variant region"

<400>  92
ctgcagaata ataaaacgcc tcatgcgatt gccgcaatta gtatggcaaa ctaactagtt    60

aagcttagcc cgccatgcat                                                 80


<210>  93
<211>  80
<212>  DNA
<213>  artificial sequence

<220>
<223>  /note="Description of artificial sequence: toxin B subunit
      variant region"

<400>  93
ctgcagaata ataaaacgcc tcatgcgatt gccgcaattg aaatggcaaa ctaactagtt    60

aagcttagcc cgccatgcat                                                 80

<210>  94
<211>  80
<212>  DNA
<213>  artificial sequence

<220>
<223>  /note="Description of artificial sequence: toxin B subunit
       variant region"

<400>  94
ctgcagaata ataaaacgcc tcatgcgatt gccgcaatta gtatgtcaaa ctaactagtt      60

aagcttagcc cgccatgcat                                                  80


<210>  95
<211>  80
<212>  DNA
<213>  artificial sequence

<220>
<223>  /note="Description of artificial sequence: toxin B subunit
       variant region"

<400>  95
ctgcagaata ataaaacgcc tcatgcgatt gccgcaattg aaatgtcaaa ctaactagtt      60

aagcttagcc cgccatgcat                                                  80


<210>  96
<211>  29
<212>  DNA
<213>  artificial sequence

<220>
<223>  /note="Description of artificial sequence: forward terminal
       primer"

<400>  96
ggcgttacta tcctctctat gtgcacacg                                        29


<210>  97
<211>  24
<212>  DNA
<213>  artificial sequence

<220>
<223>  /note="Description of artificial sequence: reverse terminal
       primer"

<400>  97
ggcgggctaa gcttaactag ttag                                             24


<210>  98
<211>  391
<212>  DNA
<213>  artificial sequence

<220>
<223>  /note="Description of artificial sequence: synthetic nucleic acid
       molecule comprising toxin B subunit variant regions"

<400>  98
atgcatacca caacacacaa atacatacgc taaatgataa gatattttcg tatacagaat      60

ctgcaggagc tcggagctcc tcaaaatatt actgatttgt gtgcagaata ccacaactgc 120

agtatacaga atctttggca ggaaaaagag agatggctat cattactttt aagaatggtg 180

caacttttct gcagtaccag gtagtcaaca tatagattca caaaaaaaag cgatagaaag 240

gatgaaggat gcatccatac acataacttt tcgactttag cttcagtaag atatgcaatc 300

ctcagggtat ctgcaggaat aataaaacgc ctcatgcgat tgccgcaatt agtatggcaa 360

actaactagt taagcttagc ccgccatgca t 391

<210> 99
<211> 274
<212> DNA
<213> artificial sequence

<220>
<223> /note="Description of artificial sequence: synthetic nucleic acid
molecule comprising toxin B subunit variant regions"

<400> 99
atgcatacca caacacacaa atacatgaac taaatgataa gatattttcg tatacagaat 60

ctgcagtata cagaatcttt ggcaggatac agagagatgg ctatcattac ttttaagaat 120

ggtgcaactt ttctgcagat accctgaggg ctgcatatct tactgaagct aaagtcgaaa 180

agttatgtgt atggatgcat ggcgggctaa gcttaactag ttagtttgcc atttcaattg 240

cggcaatcgc atgaggcgtt ttattattct gcag 274

<210> 100
<211> 130
<212> DNA
<213> artificial sequence

<220>
<223> /note="Description of artificial sequence: synthetic nucleic acid
molecule comprising toxin B subunit variant regions"

<400> 100
atgcatacca caacacacaa atacatacgc taaataagaa gatattttcg tatacagaat 60

ctgcagtata accctgagga ttgcatatct tactgaaatt aaagtcgaaa agttatgtgt 120

atggatgcat 130

<210> 101
<211> 200
<212> DNA
<213> artificial sequence

<220>
<223> /note="Description of artificial sequence: synthetic nucleic acid
molecule comprising toxin B subunit variant regions"

<400> 101
atgcatacca caacacacaa atacatgaac taaataagaa gatattttcg tatacagaat 60

ctgcagtata cagaatcttt ggcaggatac agagagatgg ctatcattac ttttgcgaat 120

ggtgcaactt ttctgcagat accctgaggg ctgcatatct tactgaaatt aaagtcgaaa 180

agttatgtgt atggatgcat                                                200

<210>  102
<211>  202
<212>  DNA
<213>  artificial sequence

<220>
<223>  /note="Description of artificial sequence: synthetic nucleic acid
       molecule comprising toxin B subunit variant regions"

<400>  102
atgcatacca caacacacaa atacatacgc taaatgataa gataaagtcg tatacagaat      60

ctgcagatac cctgaggatt gcatatctta ctgaagctaa agtctctaag ttatgtgtat     120

ggatgcatgg cgggctaagc ttaactagtt agtttgacat actaattgcg gcaatcgcat     180

gaggcgtttt attattctgc ag                                              202

<210>  103
<211>  202
<212>  DNA
<213>  artificial sequence

<220>
<223>  /note="Description of artificial sequence: synthetic nucleic acid
       molecule comprising toxin B subunit variant regions"

<400>  103
atgcatacca caacacacaa atacatgaac taaatgataa gataaagtcg tatacagaat      60

ctgcagatac cctgagggct gcatatctta ctgaagctaa agtctctaag ttatgtgtat     120

ggatgcatgg cgggctaagc ttaactagtt agtttgacat ttcaattgcg gcaatcgcat     180

gaggcgtttt attattctgc ag                                              202

<210>  104
<211>  128
<212>  DNA
<213>  artificial sequence

<220>
<223>  /note="Description of artificial sequence: synthetic nucleic acid
       molecule comprising toxin B subunit variant regions"

<400>  104
atgcatacca caacacacaa atacatacgc taaataagaa gataaagtcg tatacagaat      60

ctgcagatac cctgaggatt gcatatctta ctgaaattaa agtctctaag ttatgtgtat     120

ggatgcat                                                              128

<210>  105
<211>  128
<212>  DNA
<213>  artificial sequence

<220>
<223>  /note="Description of artificial sequence: synthetic nucleic acid
       molecule comprising toxin B subunit variant regions"

<400>  105

atgcatacca caacacacaa atacatgaac taaataagaa gataaagtcg tatacagaat 60

ctgcagatac cctgagggct gcatatctta ctgaaattaa agtctctaag ttatgtgtat 120

ggatgcat 128

<210> 106
<211> 29
<212> DNA
<213> artificial sequence

<220>
<223> /note="Description of artificial sequence: forward terminal
      primer"

<400> 106
ggcgttacta tcctctctat gtgcacacg 29

<210> 107
<211> 24
<212> DNA
<213> artificial sequence

<220>
<223> /note="Description of artificial sequence: reverse terminal
      primer"

<400> 107
ctaactagtt aagcttagcc cgcc 24

<210> 108
<211> 12
<212> PRT
<213> artificial sequence

<220>
<223> /note="Description of artificial sequence: HPV antibody binding
      sequence"

<400> 108

Leu Asp Leu Gln Pro Glu Thr Thr Asp Leu Tyr Cys
1               5                   10

## Description

The present invention relates to a method for generating an RNA library or a (poly)peptide library comprising the steps of: (a) providing one or more nucleic acid molecules each comprising i) two or more coding elements (A) each giving rise to an RNA molecule upon transcription and/or a (poly)peptide upon transcription and translation; and ii) linking elements (B) arranged according to the general formula of B(AB)₂₊ₙ, wherein said linking elements comprise one or more sequence motifs not found in said two or more coding elements allowing specific disruption of the linking elements (B); (b) cloning the nucleic acid molecule of step (a) into a vector; (c) transforming a host cell with the vector obtained in step (b) and propagating said transformed cell; (d) preparing vector DNA from the transformed and propagated cells of step (c); (e) (i) disrupting the vector DNA obtained in step (d) with one or more agents recognizing said one or more sequence motifs of the linking elements or (ii) performing an amplification step with the vector DNA obtained in step (d) and primers hybridizing to the sequence of said linking elements so that the sequences comprising the coding elements (A) are specifically amplified; (f) cloning the resulting coding elements (A) of step (e) into vectors; (g) transforming the vectors obtained in step (f) into host cells and establishing clonal colonies; and (h) culturing said clonal colonies under conditions suitable to express the coding elements. Also, the method relates to an RNA library or a (poly)peptide library obtainable or obtained according to the method of the invention. Moreover, the invention relates to a method for identifying a (poly)peptide epitope recognized by an antibody or a (poly)peptide-binding compound and a method for identifying a (poly)peptide epitope recognized by antibodies in serum. Further, the invention relates to a method for generating protein variants. Finally, the invention relates to a nucleic acid molecule as used in the method of the invention, a vector comprising the same, a cell comprising said nucleic acid molecule or said vector and a kit comprising one or more items selected from the group of said nucleic acid molecule, said vector, said cell, said RNA or a (poly)peptide library of the invention and, optionally, instructions for use.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

In the mapping of adaptive immune responses to specific proteins (antigens) a variety of different methods have been employed. In all cases the assays depend upon a form of the antigen that stimulates the response of interest. The response may be production of antibodies by B cells or the stimulation of T cells that may result in a number of different outcomes ranging from antibody production (by stimulation of B cells) and cell-mediated inflammatory responses (inducing the production of different types of cytokines depending upon the nature of the response) on one hand to suppressive responses that dampen inflammation on the other. For any given antigen an immune response is triggered by only small motifs present in the antigen. These motifs are called epitopes. In a normal immune response, antibodies are produced by specific B cells. Antibodies can recognize linear epitopes that consist of a (poly)peptide sequence and appear not to be dependent upon any higher structure that the protein may assume, but only upon the primary amino acid sequence. Other antibodies may recognize structural motifs that are entirely dependent upon higher protein structures. Such structures are called conformational epitopes and cannot be defined solely by a primary amino acid sequence. The production of antibodies in an adaptive immune response is normally dependent upon T helper (Th) cells which also have a variety of other roles in orchestrating immune responses to specific antigens. These Th cells can be divided into different types depending upon the type of response they elicit. However, they are all activated by epitopes in the antigen. These epitopes which are distinct from B cell epitopes, are linear in nature (but may contain modified amino acids) and activate T cells when they are presented to receptors on the T cells' surface by specific antigen presenting cells (APCs) in the form of a complex formed by specialized presentation proteins called the major histocompatibility complex (MHC).

Mapping of the epitopes that give rise to specific adaptive immune responses has become essential in the elucidation of mechanisms of adaptive immunity in all its myriad forms. From the structures recognized by antibodies to the epitopes that activate T cells, the protein structures that contribute to a protein's antigenicity acquire central significance, not only in helping to develop our understanding of mechanisms of adaptive immunity, but also for the development of novel vaccines and therapies based on modulation of the natural immune response.

Mapping of B cell epitopes is of importance in a variety of contexts, not least in defining the exact specificities of antibodies raised against antigens derived either from pathogens or from autoimmune responses that are indicative of different disease states. This in turn has led to concepts such as rational vaccine design based on specific pathogen-derived antigens and the development of antigen and antibody-based diagnostic analysis in which antibodies produced in response to a specific infection (that may or may not be protective) are detected. Antibodies can also be raised against specific proteins or even (poly)peptide epitopes that are present as a result of disease and incorporated into diagnostic assays.
For understanding of the basis of antibody specificity and the determination of the exact structures within a (poly)peptide epitope that are recognized, systems based upon extremely large libraries of random (poly)peptides have been developed. These (poly)peptide libraries are displayed primarily on the surface of filamentous bacteriophages which have been developed specifically for this purpose. The (poly)peptides are generated as fusion proteins in which they are linked to a protein that is exposed on the surface of the bacteriophage (Wang L-F, Yu M. Epitope Identification and Discovery Using Phage Display Libraries: Applications in Vaccine Development and Diagnostics. Current Drug Targets (2004) 5: 1-15).
(Poly)peptide display libraries can be screened on the basis of affinity of the expressed (poly)peptide tag for the antibody being analyzed. In a standard panning experiment the library is presented to the antibody of interest bound to a solid matrix. Phages carrying peptides with high affinity for the antibody are captured whereas those with little or no affinity are washed away. The bound phages are then eluted under mild conditions, amplified in bacteria and then used in further rounds of panning. Eventually phages carrying a clear consensus sequence are obtained. The technique has also been extended to express single chain antibodies and affibodies on the bacteriophage surface (Sheets MD, Amersdorfer P, Finnern R, Sargent P, Lindquist E, Schier R, Hemingsen G, Wong C, Gerhart JC and Marks JD (1998) Efficient construction of a large nonimmune phage antibody library: the production of high-affinity human single-chain antibodies to protein antigens. Proc Natl Acad Sci USA 95, 6157-6162.. Friedman M, Nordberg E, H6id6n-Guthenberg I,Brismar H, Adams GP, Nilsson FY, Carlsson J, Ståhl S. Phage display selection of Affibody molecules with specific binding to the extracellular domain of the epidermal growth factor receptor. Protein Engineering, Design Selection (2007) 20 :189-199,) and to use diverse libraries to search for molecules with high affinities for particular antigens or ligands. Generally the approach is useful when searching for peptides or proteins with affinities for specific ligands that can be provided in a pure or homogeneous form.
The general methods used for phage display require that an antibody or ligand is highly purified so that background affinities are kept to a minimum and the specificity of the approach is dependent upon this condition being met. In the case of B cell epitopes mapping is only really possible for monoclonal antibodies. These are raised against a specific antigen after a mouse has been immunized. There is therefore a lot of prior knowledge about the target protein including its primary structure. Even when antibodies are raised against complex mixtures, the broad specificity of monoclonal antibodies generated is usually determined once individual antibodies have been obtained. Only then can epitope mapping be attempted. An advantage of the method is that the random (poly)peptides will allow identification of a consensus even if the antigen is unknown. However, the approach cannot be used for analysis of polyclonal responses of purified antibodies or crude serum since other antibodies present in the mouse will react with many other (poly)peptides apart from those of interest. There are cases in which this primary polyclonal response is of interest; for example, in detection of a dominant B cell epitope.
When a more specific targeting of the displayed (poly)peptides is required the generally used method is to shotgun clone random fragments from DNA encoding the protein of interest into a phage display vector. The DNA fragments are generated by sonic disruption of the DNA fragment or by limiting digestion with DNase 1. In this case the number of different clones is much reduced but even so there is no real control over the degree of overlap, the size of the cloned fragments or the reading frame of the inserts. Therefore only a small fraction of the resulting clones are relevant or useful and consequently large libraries have to be generated to take these factors into account and again the antibodies or ligands have to be highly purified. Identification of a consensus sequence is therefore based on chance and cannot be approached based on a rational design.

The main advantage of the phage display system in (poly)peptide mapping is derived from the extreme diversity of the starting library and assumes sufficiently high affinity of the epitope for its corresponding antibody. There are, however, disadvantages. For example, as pointed out before, not all antibodies recognize linear (poly)peptide epitopes; they may recognize conformational features that cannot be duplicated with (poly)peptides.
Another more serious defect in the system is that the display systems themselves are unstable. The extreme complexity of the starting library is significantly reduced in even a small number of amplifications. This is due to the nature of the vectors used. The phage vectors express the surface protein fusions constitutively. In the case of the more common libraries the protein to which the (poly)peptide is usually fused is the gill protein product.
This protein is responsible for binding the phage to the F pilus receptor used by the phage to infect the host cell. Although the added (poly)peptide does not completely abrogate binding, it will certainly have an impact, the degree of which is determined by its size and nature. Thus a significant proportion of the population will have a disadvantage or an advantage when compared to other clones in the same library.

In the case of T-cell epitopes such extremely diverse phage display libraries are not of any use. It is still uncertain whether phages can be used as delivery particles for presentation of T cell epitopes, but even if the (poly)peptides are fused to a suitable carrier protein, there would be no way of using such a library to identify specific T cell epitopes. However, the use of (poly)peptides for this sort of mapping is potentially more broadly applicable due to the nature of T cell epitopes which are defined by primary (poly)peptide structure.
When there is a single antibody or ligand to which a binding sequence is sought the use of large libraries is advantageous, but when there are a large number of antibodies or T cells with different specificities and it is necessary to identify antibody responses to a single protein and at the same time map the specific epitopes present on the protein, it would be better to use a library composed of sequences specific for the protein of interest. Indeed, this principle is already used in the mapping of T cell epitopes.
T cell epitopes are linear (poly)peptides derived from the protein against which the response is directed. In order to activate T cells the protein has to been taken up and processed by an APC. This involves the proteolytic cleavage of the protein into small (poly)peptides, some of which are then incorporated into a presentation complex and transported back to the cell surface. Specific T cells, via a receptor on their surface (the T cell receptor) recognize and bind to the complex containing the correct (poly)peptide. This interaction together with the interaction of other co-stimulatory molecules results in the activation of the T cell triggering rapid cell division and the release of cytokines. The basis of assays of T cell activation is to either follow the expansion of T cells following addition of the antigen in the presence of APCs or to follow the production of specific cytokines. All the assays use cell culture and are therefore considerably more expensive and time consuming than the mapping of B cell epitopes. Critically, the ability to recover and expand populations of phages following elution from the antibodies to which they bind in the case of B cell epitopes is not applicable in the case of T cell assays. The antigen is necessarily destroyed in the process of T cell activation. Thus a different strategy has to be employed.
Mapping of T cell epitopes and also the broad and less specific mapping of B cell epitopes is done using synthetic (poly)peptides. The (poly)peptides represent defined segments of the protein in question and contain varying degrees of overlap in order to cover the maximum number of possible epitopes. For large proteins in which a large degree of overlap is required, this can result in a very large number of (poly)peptides. This in turn leads to a question of economics since the price of such (poly)peptides can become prohibitive, and when consumed, they have to be re-synthesized. In general this consideration leads to a number of compromises and T cell epitopes tend to be localized to relatively long (poly)peptides relative to the size of the minimal epitope sequence. The processing of the (poly)peptide by the different proteases that result in the (poly)peptide that is loaded into the presentation complex is also affected by the flanking regions.

In summary, presently available methods using (poly)peptide libraries, for example in epitope mapping as outlined above, are hampered in many ways as regards the size of the library, the confirmation of a minimal epitope consensus sequence, the control over the size and nature of the (poly)peptides to be screened, the reproducibility of the library and finally the monetary burden associated with establishing and maintaining a (poly)peptide library in various complexities.

The technical problem underlying the present invention was to identify alternative and/or improved means and methods to generate (poly)peptide libraries.

The solution to this technical problem is achieved by providing the embodiments characterized in the claims.

Accordingly, the present invention relates in a first embodiment to a method for generating an RNA library or a (poly)peptide library comprising the steps of:
(a) providing one or more nucleic acid molecules each comprising
   i) two or more coding elements (A) each giving rise to an RNA molecule upon transcription and/or a (poly)peptide upon transcription and translation; and
   ii) linking elements (B)
      arranged according to the general formula of B(AB)₂₊ₙ, wherein said linking elements comprise one or more sequence motifs optionally comprising restriction endonuclease recognition sites not found in said two or more coding elements allowing for specific disruption of the linking elements (B);
(b) cloning the nucleic acid molecule of step (a) into a vector;
(c) transforming a host cell with the vector obtained in step (b) and propagating said transformed cell;
(d) preparing vector DNA from the transformed and propagated cells of step (c);
(e) (i) disrupting the vector DNA obtained in step (d) with one or more agents recognizing said one or more sequence motifs of the linking elements or (ii) performing an amplification step with the vector DNA obtained in step (d) and primers hybridizing to the sequence of said linking elements so that the sequences comprising the coding elements (A) are specifically amplified;
(f) cloning the resulting coding elements (A) of step (e) into vectors;
(g) transforming the vectors obtained in step (f) into host cells and establishing clonal colonies; and
(h) culturing said clonal colonies under conditions suitable to express the coding elements.

The term "(poly)peptide" accordance with the present invention describes a group of molecules which comprises the group of peptides, consisting of up to 30 amino acids, as well as the group of polypeptides, consisting of more than 30 amino acids. Also encompassed by the term "polypeptide" are fragments of polypeptides (if longer than 30 amino acids; otherwise those fragments are called "peptides"). The term "fragment of a polypeptide" in accordance with the present invention refers to a portion of a polypeptide comprising at least the amino acid residues necessary to maintain the biological activity of said polypeptide. (Poly)peptides may further form dimers, trimers and higher oligomers, i.e. consisting of more than one (poly)peptide molecule. (Poly)peptide molecules forming such dimers, trimers etc. may be identical or non-identical. The corresponding higher order structures are, consequently, termed homo- or heterodimers, homo- or heterotrimers etc. The terms "polypeptide" and "protein" is herein used interchangeably. The term "(poly)peptide" also refers to naturally modified (poly)peptides where the modification is effected e.g. by glycosylation, acetylation, phosphorylation and similar modifications. Said modifications and methods to artificially introduce them are well-known in the art.

The nucleic acid molecule employed in the method is structurally defined by the presence of two or more coding elements and linking elements arranged according to the formula B(AB)₂₊ₙ, wherein "n" may be any positive integer or zero. Hence, in its minimal form an RNA library or (poly)peptide library consists of only two RNA molecules or (poly)peptides. Preferably, the libraries of the invention are more complex. Therefore, it is envisaged that "n" is a positive integer in the range of 1 to 5000 such as 5, 10, 50, 100, 200, 300, 400, 500, 600, 700, 800, 900, 1000, 2000, 3000, 4000 or any deliberate positive integer in that range. Complexity may also be achieved when providing more than one nucleic acid molecule in step (a) such as at least (for each of the following) 10, 10¹, 10², 10³, 10⁴, 10⁵, 10⁶, 10⁷ or at least 10⁸.

The term "coding elements" relates to a nucleic acid sequence such as, e.g., (genomic) DNA or cDNA, that gives rise to either an RNA molecule upon transcription and/or a (poly)peptide upon transcription and translation , i.e. the coding elements are expressed. "Expression" relates to a process by which information from a nucleic acid sequence such as, e.g., a gene or a gene fragment, is processed into a genetic product such as, e.g., an RNA molecule or a (poly)peptide. Said process can be subdivided into a transcriptional and a translational process. Transcription describes in the context of gene expression the process of transcribing DNA into an RNA molecule such as, e.g., mRNA, tRNA or miRNA, whereas translation describes the process of translating mRNA into a (poly)peptide. Preferably, the sequence of the coding elements is different, i.e. coding, e.g., for different (poly)peptides and/or mutants/allelic variants of the same (poly)peptide. It is envisaged that the two or more coding elements may exclusively code for RNA molecules which are not translated into (poly)peptides or encode (poly)peptides. A coding element in accordance with the invention may comprise any naturally or non-naturally occurring sequence. Preferably, the sequence comprises or consists of one or more sequence stretches annotated with a specific effect or function. Said sequence stretches may comprise or consist of, e.g., one or more genes, fragments of genes, genetic elements such as, e.g., enhancer, silencer, in- or extronic regulatory or signalling sequences, protein or nucleic acid binding motifs or sequence stretches coding for (antigenic) epitopes. It is also envisaged that more than one sequence stretch with the same or different annotated function or effect be contained in a coding element. For example, two or more identical or different sequence stretches may be arranged in a repetitive manner to make up a coding element. Information on a function or effect (potentially) annotated with a sequence may be retrieved from a variety of online databases available such as, e.g., SYFPEITHI (http://www.syfpeithi.de/) or NCBI databases (http://www.ncbi.nlm.nih.gov/sites/gquery), or may be determined *in silico* with computer-aided programs that allow a prediction of function or effect of a nucleic or amino acid sequence based upon said sequence and/or structure inherent to said sequence due to intra-molecular affinities. It is particularly preferred that said coding elements encode (poly)peptides representing or comprising B cell epitopes or T cell epitopes.

The term "linking elements" as used herein relates to nucleic acid sequences linking the coding elements to each other and are also found at each terminus of a nucleic acid molecule in accordance with the invention and as summarized in the formula B(AB)₂₊ₙ. The nucleic acid sequence of a linking element comprises one or more sequence motifs which are not found in the two or more coding elements. Preferably, the nucleic acid sequence of a linking element consists only of said one or more sequence motifs. Also preferred is that the sequence of the linking elements is the same while at the same time it is envisaged that they can be different. The sequence motif may be, e.g., any motif that upon appropriate treatment of the nucleic acid molecule alone or when cloned into a vector with an agent specific for said motif results in disruption of the nucleic acid molecule of the invention resulting in single coding elements. The agent to be used for disruption is dependent on the motif used. For example, in the case of restriction endonuclease recognition sites restricition endonuclease can be use, in the case of loxP sites Cre-recombinase my be used. A sequence motif may also be a modification of the DNA that may be cleaved by the action of a chemical agent or enzyme. In a preferred embodiment, the disruption of the nucleic acid molecule of the invention alone or when cloned into a vector generates coding elements devoid of sequence fragments of linking elements. This can be achieved by the use of restriction enzymes that cleave the DNA at a site distant from their recognition site. In such cases the linker sequence (B) in the motif B(AB)₂₊ₙ can be removed entirely. A minimal linking element consists, e.g., of at least one restriction endonuclease recognition site or a primer binding site. Preferably, a linking element of the invention comprises or consists of two different endonuclease recognition sites as sequence motifs. Such a makeup allows for orientation-specific cloning of a coding element after digestion of the nucleic acid molecule as defined herein with restriction endonucleases recognising said two different recognition sites. At the same time it is envisaged that the linking element comprises or consists of only one endonuclease recognition site recognised by two different restriction endonucleases. Mandatory for the method of the invention is that the one or more sequence motifs, e.g., restriction endonuclease recognition sites to be used for cloning or the primer binding sites used for amplification of coding elements, are exclusively found in the linking elements. Alternatively, said sequence motifs may be masked in the coding elements, e.g., by nucleic acid modification such as methylation resulting in cleavage only at the recognition sites in the linking elements in the case of endonuclease recognition sites. Sequence motifs that may be part of a linking element in accordance with the invention are selected from the group of, e.g. restriction endonuclease recognition sites or recombinase recognition sites, promoters, transcription terminators or enhancers, ribosome binding sites. Restriction endonucleases are enzymes of archeal and bacterial origin well-known to the person skilled in the art. Restriction endonucleases catalyze the cleavage of the phosphodiester bonds within a polynucleotide chain, i.e. double or single nucleic acid strands. Cleavage occurs at specific sites termed restriction endonuclease recognition sites. Commonly, restriction endonucleases are classified into three families, Type I, II and III based upon their mechanism of action. Recognition sites vary in sequence and length from type to type even from enzyme to enzyme. A common feature of endonuclease restriction sites is a palindromic sequence. There are mirror-like palindromes as well as inverted-repeat palindromes, wherein the latter occurs more often. Cleavage of the DNA by restriction endonucleases can result in DNA-termini with overhangs ("sticky ends") or without ("blunt ends"). While most restriction endonucleases have their own specific and exclusive recognition sequence, some endonucleases share the same recognition site (neoschizomers) but may cleave at different sites within said recognition sequence. Also, additional nucleic acid bases besides the nucleic acid bases making up the sequence motifs described above may be part of a linking element as spacers, e.g., to adjust the reading frame when the nucleic acid molecules of the invention are cloned into a vector in order to express the coding elements in the native reading frame in the case a (poly)peptide library is to be generated. It is also envisaged that upon cleavage of element (B), all nucleotides belonging to linking elements are removed from the coding elements (A).

The above described coding element (A) and linking element (B) make up a continuous sequence of the nucleic acid molecule defined herein according to the formula B(AB)₂₊ₙ. The two or more coding elements may be homogeneous/identical or heterogeneous/different regarding their length or nature of origin. The same holds true also for the linking elements, which accordingly may comprise or consist of the identical sequence or have a different sequence. Since the coding elements may have varying or equal sizes the linking elements - also being homogeneous or heterogeneous - will link said coding elements either in a constant or varying pattern. In other words, the specific makeup of the single coding and linking elements dictates the ultimate makeup of the nucleic acid molecule as claimed. Preferably, said nucleic acid molecule is produced synthetically. Methods for synthetic production of nucleic acids of a desired sequence are well-known in the art. Alternatively, the respective desired nucleic acid sequences may be if naturally occurring be isolated from a suitable host and combined to yield a nucleic acid molecule as described above. Methods for isolation of nucleic acid sequences from organisms and fusion are well-known in the art and inter alia described in Sambrook and Russell, "Molecular Cloning, A Laboratory Manual", Cold Spring Harbor Laboratory, N.Y. (2001).

The term "cloning" is well-known in the art and different methods are described in a multitude of textbooks and scientific articles. A number of cloning method variations are without limitation described, e.g., also in Sambrook and Russell (2001).

The term "vector" is equally well-known to the skilled person in the art and also described, e.g., in Sambrook and Russell. In accordance with the invention, the vector is a plasmid, cosmid, virus, bacteriophage or another vector used e.g. conventionally in genetic engineering.

The nucleic acid molecule of the present invention may be inserted into several commercially available vectors. Non-limiting examples include prokaryotic plasmid-vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1 neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen).

The nucleic acid molecule of the present invention referred to above may also be inserted into vectors such that a translational fusion with another nucleic acid molecule is generated. The other nucleic acid molecule may encode a protein which may e.g. increase the solubility and/or facilitate the purification of the fusion protein, or code for a phage protein for phage display. Non-limiting examples include pET32, pET41, pET43.

For vector modification techniques, see Sambrook and Russel (2001) referred to herein above. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources. Ligation of the coding elements to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, the coding elements of the invention are operatively linked to such expression control sequences allowing expression in prokaryotes or eukaryotic cells resulting in RNA molecules and/or (poly)peptides. The vector may further comprise nucleotide sequences encoding secretion signals as further regulatory elements. Such sequences are well known to the person skilled in the art. Furthermore, depending on the expression system used, leader sequences capable of directing the expressed (poly)peptide to a cellular compartment may be part of the coding sequence of a vector. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1α-promoter, CMV enhancer, CaM-kinase promoter, the Autographa californica multiple nuclear polyhedrosis virus (AcMNPV) polyhedral promoter or the SV40-enhancer. For the expression in prokaryotes, a multitude of promoters including, for example, the tac-lac-promoter, the lacUV5 or the trp promoter, has been described. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

Furthermore, it is preferred that the vector of the invention or any vector to be used in the method of the invention comprises a selectable marker. Examples of selectable markers include neomycin, ampicillin, and hygromycin resistance and the like. Specifically-designed vectors allow the shuttling of DNA between different hosts, such as bacteria- fungal cells or bacteria-animal cells.
An expression vector to be used in this invention is capable of directing the replication, and the expression, of RNA molecules and/or (poly)nucleotides that are part of the respective libraries. Suitable expression vectors which comprise the described regulatory elements are known in the art such as Okayama-Berg cDNA expression vector pcDV1 (Pharmacia), pRc/CMV, pcDNA1, pcDNA3 (In-Vitrogene, as used, inter alia in the appended examples), pSPORT1 (GIBCO BRL) or pGEMHE (Promega), or prokaryotic expression vectors, such as lambda gt11, pJOE, the pBBR1-MCS -series, pJB861, pBSMuL, pBC2, pUCPKS, pTACT1 or, preferably, the pET vector (Novagen).
The nucleic acid molecules of the invention cloned into the vectors as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention.

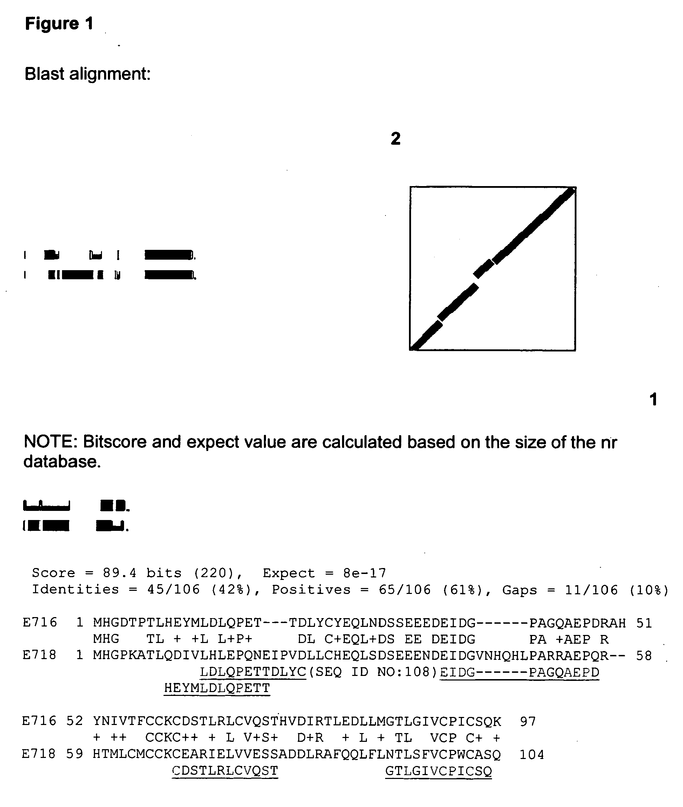

A typical mammalian expression vector contains a promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The lac promoter is a typical inducible promoter, useful for prokaryotic cells, which can be induced using the lactose analogue *isopropylthiol-b-D-galactoside.* ("IPTG"). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the RNA molecule or (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of a target sequence of interest. Another useful selection marker is the enzyme glutamine synthase (GS) (Murphy et al.1991, Biochem J. 227:277-279; Bebbington et al. 1992, BiolTechnology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli*, Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art. A preferred vector to be used and as exemplified in the Example section (cf. also Fig 1) is based on the pBAD vectors form Invitrogen, which are modified to be a phagemid that expresses the gVIII product from M13 to which peptides can be fused. It is envisioned that other vectors allowing the insertion of the same synthesis to be used to fuse (poly)peptides to other filamentous phage components (such as the gIII product) or to other carrier (poy)peptides such as 26 kDa Glutathione-s-transferase or the B subunit of cholera toxin are constructed.

The term "propagation" in relation to the culturing of cells, in particular host cells, is well-known in the art and has no other meaning in accordance with the invention. Said term relates to the amplification of cell numbers via cell division in cell culture. The person skilled in the art is well-aware of means and methods for establishing and maintaining a cell culture, such as, for example, choice of media constituents, markers, cell amplification and isolation. Corresponding methods are described in a variety of textbooks and scientific papers such as, e.g., "Epithelial Cell Culture Protocols", edited by C. Wise, Vol. 188, ISBN: 978-0-89603-893-6, Humana Press; "Practical Cell Culture Techniques", Boulton et Baker (eds), Humana Press (1992), ISBN 0896032140; "Human Cell Culture Protocols", Gareth E. Jones, Humana Press (1996), ISBN 089603335X. Growth media and other cell culture related material as well as instructions and methods for successful culturing of cells can, for example, be obtained at Sigma-Aldrich or Invitrogen. Generally, cells are grown and maintained at an appropriate temperature and gas mixture, i.e. typically 37°Celsius, 5% CO₂ in growth media (a) as irrigating, transporting and diluting fluid while maintaining intra- and extra-cellular osmotic balance, (b) that provides cells with water and certain bulk inorganic ions essential for normal cell metabolism, (c) which - combined with a carbohydrate, such as glucose - provides the principle energy source for cell metabolism and (d) which provides a buffering system to maintain the medium within physiologic pH range, i.e. cells are kept viable. The recipe of growth media varies greatly depending on cell-type and contains, for example and without limitation, growth factors, nutrient components, glucose, buffers to maintain pH and antifungizides and -biotics. The person skilled in the art is aware of cell culture conditions suitable to enhance expression or proliferation of cultured cells. Corresponding methods are equally described in the above cited references regarding cell culture methods.

Digestion of vector DNA with restriction endonucleases is a standard procedure in molecular cell biology. Briefly, (vector) DNA is purified in order to eliminate factors that may inhibit the enzyme activity of the restriction endonucleases and contacted with said endonucleases in a buffered solution, optionally comprising further additives to enable or enhance catalytic activity. Suitable reaction conditions vary from enzyme to enzyme and are usually referred to in instructions for use when the endonucleases are ordered from a commercial vendor. If a sequence is to be digested with more than one restriction endonuclease this can be done serially or simultaneously. However, serial digestion may require purification of the nucleic acid sequence and/or adaptation of the reaction conditions prior to the digestion with a further endonuclease. Simultaneous digestion only works with specific compatible endonucleases. Since methods for digesting DNA are well-known in the art the skilled person is in the position to select endonucleases and perform digests according to his specific needs taking into account potential fine-tuning of the reaction conditions depending on the restriction endonucleases used.

The term "hybridizing" is well-known in the art with regard to nucleic acid sequences and as used herein refers to pairing of a polynucleotide, e.g., a primer, to a preferably completely complementary strand of another polynucleotide which thereby form a hybrid. At the same time it is also envisaged in accordance with the invention that hybridization may occur between two not completely complementary strands. For example, a 1, 2, 3, 4 or more base pair mismatch may exist and consequently the hybridization can take place only partially.

It is well known in the art how to hybridize polynucleotide sequences and how to perform hybridization experiments with polynucleotide sequences. Accordingly, the person skilled in the art knows what hybridization conditions she/he has to use to allow for a successful hybridization in accordance with item (i)(c), above. The establishment of suitable hybridization conditions is referred to in standard text books such as Sambrook and Russell; Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).

The term "amplification" or "amplify" means increase in copy number. The person skilled in the art know various methods to amplify polynucleotide sequences, these methods may also be used in the present invention's methods. Amplification methods include, but are not limited to, "polymerase chain reaction" (PCR), "ligase chain reaction" (LCR, EPA320308), "cyclic probe reaction" (CPR), "strand displacement amplification" (SDA, Walker et al. 1992, Nucleic Acid Res. 7: 1691-1696), "transcription based amplification systems" (TAS, Kwoh et al. 1989, Proc. Nat. Acad. Sci. USA 86: 1173; Gingeras et al., PCT Application WO 88/10315). Preferably, amplification of DNA is accomplished by using polymerase chain reaction (PCR) [Methods in Molecular Biology, Vol. 226 (Bartlett J. M. S. & Stirling D., eds.): PCR protocols, 2nd edition; PCR Technology: Principles and Applications for DNA Amplification (Erlich H. A., ed.), New York 1992; PCR Protocols: A guide to methods and applications (Innis M. A. et al., eds.), Academic Press, San Diego 1990]. Nucleic acid amplification methods may be particularly useful in cases when the sample contains only minute amounts of nucleic acid. If said nucleic acid is RNA, an RT-PCR might be performed. Subsequently, another amplification step involving PCR may be performed. Alternatively, if said nucleic acid contained in the sample is DNA, PCR may be performed.
Amplification of nucleic acid sequences is preferably performed by polymerase chain reaction (PCR) with suitable primers. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl2, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix.
Primers can be designed by methods well-known in the art including, for example, publicly available programs as primer3 (http://frodo.wi.mit.edun. Preferred primers to be used in the methods of the invention are primers that completely hybridize, i.e. no mismatch occurs, to target sequences of the nucleic acid molecules of the invention.

"Establishing clonal colonies" is a procedure well-known in the art and relates to singling out colonies of host cells that have been successfully transformed with the vectors comprising the coding elements described herein above. This can be achieved by using selection markers as described herein above being part of the introduced DNA sequences, preferably vectors. "transforming" cells relates to the procedure of introducing polynucleotide sequences into cells. Means and methods to transform cells are well-known in the art and described, e.g., in Sambrook and Russell referred to above.

In a further embodiment, the invention relates to a variation of the above described method differing in that the nucleic acid molecule is not cloned into a vector for amplification but is instead amplified as a whole using primers specifically recognizing the termini of the one or more nucleic acid molecules of the invention. Subsequently, the amplified nucleic acid molecules are processed the same way as described in the main embodiment. In a further variation of the main embodiment steps (b) to (e) are replaced by an initial amplification step with primers that recognize a sequence motif in the linking elements and result in amplification of each coding element which then, as a single coding element, can be cloned into a vector as in step (f), the vector transformed into host cells as in step (g) and finally as in step (h) of the main embodiment clonal colonies are cultured to express the coding elements.

The design of the coding elements, in particular when using fragments of a gene or another sequence annotated with a function or effect as coding elements, as well as the linker sequences and the overall design of the nucleic acid molecule of the invention is preferably effected *in silico.* Corresponding bioinformatical programs that may or may not be used in connection with online databases are well-known to the person skilled in the art.

The above-described invention is to some extent taking advantage of the rapidly evolving technology surrounding the synthesis of synthetic genes. Whereas it has to date not been possible to synthesize whole proteins, the size of DNA fragments that can be synthesized has increased dramatically in recent years making it possible to chemically synthesize entire genes and the prospect of entire pathways and indeed entire genomes is no longer considered to be out of reach. Putting the above described invention into practice (as described in the example section below), the inventors have been able to generate a renewable (poly)peptide library. Briefly, a (poly)peptide library used to map monoclonal and polyclonal antibodies was generated by designing and synthesizing a nucleic acid molecule as described herein above of the formula B(AB)₂₊ₙ. The nucleic acid molecule was rationally designed to contain defined overlapping fragments of the gene of interest. The fragments make up the coding elements of a nucleic acid molecule being linked by linker elements containing restriction endonuclease sites. Said nucleic acid molecule was synthesized and cloned for amplification into host cells. After digestion the resulting single coding elements were re-cloned into host cells for amplification and clonal cell lines were established each containing a specific protein encoded by the fragment, i.e. the coding element. The (poly)peptides produced were used in mapping assays as detailed below in the example section. As becomes immediately evident, the advantage of the method described herein is that products can be amplified and are essentially renewable without costly repetitions of chemical syntheses. They can be used in much the same way as any other phage display library to identify specific binding affinities, but the banks contain far fewer and exactly predetermined - if desired - sequences and the chances of success are therefore significantly increased.
A further advantage of the present invention, following immunization, crude sera (cf. example 1) can be screened for immune responses to a specific protein and at the same time relevant epitopes can be mapped. This significantly reduces the amount of work required for epitope mapping and can be useful for example in epidemiological studies of immune responses to vaccine antigens or due to infections. Phage-linked antigens are at present not particularly useful for screening proteins for T cell epitopes. However, with limited numbers of defined overlapping (poly)peptides from a specific protein, this task can be made much easier. The DNA encoding the (poly)peptide library for a specific gene can be cloned into fusion protein vector and expressed as a library of (poly)peptides attached to a convenient carrier protein such as the 26 kDa gluthione-S-transferase from *Schistosoma japonicum* or linked to the B subunit of cholera toxin. Such (poly)peptide libraries can be used in T cell stimulation assays. As is described, the size of a library can be varied to contain different numbers of (poly)peptides and/or different regions from a given (poly)peptide. Having identified a group of (poly)peptides with stimulating activity encoded by coding elements, subsets of the library can be screened for the individual (poly)peptides responsible. In order to make maximum use of a single synthesis, expression vectors are designed so that the same (poly)peptides are part of a phage display library or a fusion protein expression library.
As one of the possible applications (besides, e.g., T- or B-cell mapping) of the present invention, it is envisaged that proteins that are known to bind to each other (e.g., part of a protein-protein-interaction chart), e.g., the binding has been determined by, e.g., a yeast-two-hybrid assay or *in silico* binding studies, are further analyzed for the specific sequence within the native protein that mediates binding. Preferably, each binding partner, i.e. each native protein, is fragmented on the DNA level into suitable sequence fragments to be part of the coding elements as described herein each resulting in a library comprising exclusively of (poly)peptides from one binding partner. Subsequently, the (poly)peptides of one library may be subjected to binding studies with one or more (poly)peptides of the other library in order to identify the minimal sequences responsible for binding of the native proteins.

Also envisaged in a possible application of the present invention is the coupling of (poly)peptides generated according to the method of the invention to microbodies^{™}, i.e. they are used as carriers. "Microbodies^{™}" are very small and stable (poly)peptides (about 28 to 45 amino acid residues) with pseudo-knot structures that contain 3 to 4 cystein bridges. The structure of microbodies allows for the insertion of binding (poly)peptide sequences into their loops. Preferably, said peptides are 5 to 20 amino acids long. Microbodies and methods for coupling to other (poly)peptides have been described in, e.g., US 7,186,524 B2. Since microbodies are very small and exhibit protease-resistance, they can be used as carrier molecules for (poly)peptides of a library generated according to the method of the present invention that, e.g., are to be administered to a subject/patient in a therapeutic setting.

In a preferred embodiment, the method of the invention further comprises after step (h) the additional step (i) of isolating the RNA molecule and/or (poly)peptide encoded by the coding element of each clonal colony.

Kits for isolation and/or purification of RNA molecules are commercially available and methods for the isolation of (poly)peptides are well-known in the art.
The libraries of the present invention may also consist of isolated RNA molecules and (poly)peptides thereby being readily accessible for applications since the step of propagating clonal colonies and expressing coding elements to obtain said RNA and/or (poly)peptides is obsolete.

In another preferred embodiment of the method of the invention, the two or more coding elements when arranged as a continuous sequence comprise the entire or partial coding sequence of one or more genes in the native reading frame.

The entire or partial coding sequence of a gene may be divided into a desired number of fragments each of which is a coding element. The coding elements may be assembled in any order in the nucleic acid molecule of the invention, i.e. need not be arranged according to their position respective to the unfragmented entire or partial gene. The coding elements each comprising or consisting of a fragment are connected to the linking elements in the nucleic acid molecule so that the sequence of the linking element encodes, e.g., one or more in frame restriction endonuclease recognition sites or sequence motifs specifically recognized by PCR-primers, so that the nucleic acid molecule can be digested resulting in single coding elements or the coding elements be amplified and cloned into a vector to allow expression of a (poly)peptide fragment in the native reading frame as described herein above.

In a more preferred embodiment of the method of the invention, the coding elements when arranged as a continuous sequence comprise overlapping sequences.

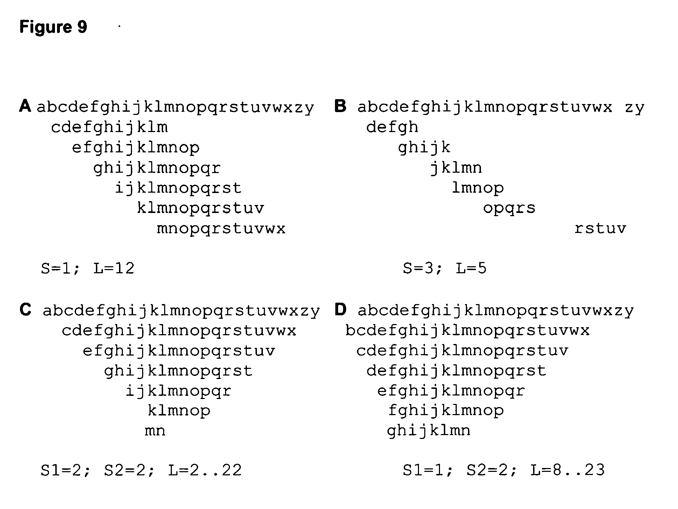

The degree of overlap may be - when expressed in percent - up to 100%, i.e. the sequences are identical. The presence of several identical coding elements may be suitable in situations where one aims at selectively increasing a specific RNA molecule or (poly)peptide in a corresponding library. However, an overlap of only one base pair is also envisaged. Any deliberate number of overlapping base pairs in the range of 1 base pair to all base pairs is expressly envisioned in accordance with the invention. The pattern of the fragments making up the coding elements of the invention can be of any design and ultimately depends on the specific experimental setup and goal to be achieved. The nucleic acid molecule of the invention may comprise overlaps of different lengths either growing or shrinking giving a symmetric or asymmetric pattern or a di-centric pattern of overlaps in view of the starting sequence or a random pattern of overlaps and non-overlapping or only partially overlapping fragments (cf. Fig. 9). In order to suppress potential recombination between homologuous regions of the nucleic acid molecule and to improve its clonability, silent mutations, i.e. mutations not changing the encoded amino acid sequence, may be introduced on the nucleic acid level.

The above described structure of the coding elements comprising overlapping sequences when arranged as a continuous sequence proves particularly advantageous in generating scanning libraries, wherein the overlapping sequences are overlapping fragments of one starting sequence such as, e.g., a gene or part of a gene. The RNA molecules or (poly)peptides encoded by the coding sequences may subsequently be employed in functional screenings such as, e.g., a T-cell assay and general assay described in "Current protocols in Immunology", Wiley interscience, Print ISSN: 1934-3671, online ISSN: 1934-368X, suitable for the systematic analysis of minimal consensus binding sequences for any RNA or (poly)peptide binding compound such as antibodies or drugs.

In another preferred embodiment of the method of the invention the one or more coding elements comprise a sequence variant of a sequence.

A "sequence variant" of a sequence means in accordance with the invention a nucleic acid sequence that comprises a variation in its sequence in comparison to a reference nucleic acid sequence. For example, a sequence carrying a mutation is a sequence variant to its corresponding wild-type sequence. Accordingly, a sequence variant may be a sequence carrying a mutation. The variation in accordance with the invention may be manifested as a partially or completely different sequence with regard to said reference sequence. The sequence variation may be an addition, deletion or a substitution of one or more bases. Preferably, the variation is a non-silent variation, i.e. the variation changes the amino acid sequence of an encoded (poly)peptide in comparison to said reference sequence. The person skilled in the art is well-aware how to introduce non-silent and silent variations into a given nucleic acid sequence in view of his knowledge of the degeneracy of the genetic code.

This embodiment of the invention may be useful in order to diversify gene sequences, increasing complexity of a library and in subsequent functional assays identify minimal consensus and optimal binding sequences for any RNA or (poly)peptide binding compound such as antibodies or drugs.
In a different preferred embodiment of the method of the invention, the nucleic acid molecule is synthetically produced. Methods for the synthetic production of nucleic acid molecules are well-known in the art and have been described herein above.

In a further preferred embodiment of the method of the invention, the vector in step (f) is a fusion protein vector allowing the expression of a (poly)peptide encoded by a coding element fused to a phage protein for phage display. The method known as phage display as well as fusion vectors are described herein above and a specific example is given in the Example section.

In another embodiment the invention relates to an RNA library or (poly)peptide library obtainable or obtained according to the method of the invention. As is evident from the embodiments described above, a custom-made RNA or (poly)peptide library can be generated which is specifically tailored to a given experimental strategy and for use in a variety of functional assays.

Also, the invention relates to a method for identifying a (poly)peptide epitope recognized by an antibody or a (poly)peptide-binding compound comprising the steps of:
(a) preparing a (poly)peptide library according to the method of the invention;
(b) subjecting said clonal colonies of the (poly)peptide library to immunological screening with the antibody of interest to identify clonal colonies expressing a (poly)peptide that is bound by said antibody or said (poly)peptide-binding compound of interest; and optionally
(c) based on the result obtained in step (b) sequencing the coding elements of the vectors of the clonal colonies to identify the (poly)peptide epitope recognized by said antibody or said (poly)peptide-binding compound.

The passage "immunological screening" as used in accordance with the present invention relates to a procedure that results in the identification of binding between a (poly)peptide of the (poly)peptide library with an antibody or a (poly)peptide-binding compound. Various methods are known in the art to visualize said binding and are described, e.g., in Sambrook and Russell referenced above.

An "antibody" can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. These antibodies can be used, for example, for the immunoprecipitation, affinity purification and immunolocalization of the (poly)peptides or fusion proteins described herein and part of the claimed libraries as well as for the monitoring of the presence and amount of such (poly)peptides, for example, in cultures of recombinant prokaryotes or eukaryotic cells or organisms.

The term "antibody" may also comprise chimeric (human constant domain, non-human variable domain), single chain and humanized (human antibody with the exception of non-human CDRs) antibodies, as well as antibody fragments, like, inter alia, Fab or Fab' fragments. Antibody fragments or derivatives further comprise Fd, F(ab')₂, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals or plants (see, e.g., US patent 6,080,560) may be used to express (humanized) antibodies specific for the target of this invention. Most preferably, the antibody of this invention is a monoclonal antibody. For the preparation of monoclonal antibodies, any technique which provides antibodies produced by continuous cell line cultures can be used. Examples for such techniques include the hybridoma technique (K6hler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a (poly)peptide of the invention (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, amongst others, viruses or plasmid vectors.

The antibody described in the context of the invention is capable to specifically bind/interact with an epitope of the polypeptides or fusion protein of the invention. The term "specifically binding/interacting with" as used in accordance with the present invention means that the antibody does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest. Corresponding methods are described e.g. in Harlow and Lane, 1988 and 1999, loc cit.

A "(poly)peptide-binding compound" as referred to herein can be any compound that is known or suspected to bind to a (poly)peptide sequence. To evaluate whether a compound potentially binds to a (poly)peptide sequence binding studies can be performed *in vitro, ex vivo, in vivo* and *in silico.* Corresponding methods are well-known in the art such as, e.g., HPLC/MS, ELISA. Compounds known to exhibit (poly)peptide binding properties belong to the classes of, e.g., antibodies, drugs, DNA, aptamers, small organic or inorganic molecules, hormone receptors, etc.

One way of performing step (b) of this embodiment, i.e. the immunological screening, is exemplarily described in Example 1 below. Briefly, a (poly)peptide library was generated and each clonal colony comprising and expressing a specific coding element was propagated and subsequently transferred to a nitrocellulose filter and allowed to grow. Finally, the cells of the clonal colonies were lysed in situ and binding of monoclonal antibodies was visualized using an antibody conjugated to horseradish peroxidase and directed against said monoclonal antibodies. Nevertheless, also other ways of performing immunological screening are expressly envisioned such as, e.g., isolating the (poly)peptides of a library and attaching the same on a solid support for subsequent detection of binding as, e.g., in an ELISA assay. A solid support according to the invention provides a surface for the attachment of the (poly)peptides encoded by the coding elements. Said surface in accordance with the invention may be any surface. The surface may be a coating applied to the support or carrier, or the surface of the support or carrier itself may be used. Support or carrier materials commonly used in the art and comprising glass, plastic, gold and silicon are envisaged for the purpose of the present invention.

Coatings according to the invention, if present, include poly-L-lysine- and aminosilane-coatings as well as epoxy- and aldehyde-activated surfaces.

The optional step (c) of sequencing may be performed according to well-known methods used for sequencing DNA molecules. For example and without limitation, the dye termination method can be used. The methods and mechanisms underlying the dye termination method (Sanger didesoxy chain termination) are well known in the art and described (F. Sanger et al., (1977), DNA sequencing with chain-terminating inhibitors; Proc Natl Acad Sci USA, 74:5463-5467). Further, approaches of sequence analysis by direct sequencing, fluorescent SSCP in an automated DNA sequencer and Pyrosequencing are envisioned. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

The method described in this embodiment allows for screening and identifying of (poly)peptide sequences that are bound by an antibody or a (poly)peptide-binding compound. "Binding" as used in accordance with the invention refers to binding (preferably specific, i.e. not cross-reacting) to linear sequence as well as to secondary or tertiary structures inherent to the (poly)peptide of a library. As becomes evident in view of the foregoing, one can establish customized (poly)peptide libraries that allow a rational approach to screening, e.g., antibody epitopes. In particular, the resolution power may be fine-tuned on the DNA-level and hence allows for screening any desired sequence in any detail, i.e. a high resolution can be achieved by incorporating fragments into coding elements that display a high degree of overlap when arranged as a continuous sequence. In this regard, it may be possible to initially start with large fragments and upon localizing a fragment whose expression product is bound by the antibody further fragment said large fragment and continually increase the resolution power by decreasing fragment size and increasing overlap of the fragments in order to identify the minimal consensus sequence responsible for binding of the antibody.

In a further embodiment, the invention relates to a method for identifying a (poly)peptide epitope recognized by antibodies in serum comprising the steps of:
(a) preparing a (poly)peptide library according to the method of the invention;
(b) infecting the clonal colonies with a helper phage and obtaining phages carrying the fusion protein vector for each clonal colony;
(c) contacting the phages obtained in step (b) with the serum;
(d) determining binding of phages to antibodies in said serum; and optionally
(e) based on the result of step (d) sequencing the coding element of the vector of the bound phages to identify the (poly)peptide epitopes recognized by antibodies in said serum.

The terms "phage display", "fusion protein vector" have been described herein above. Moreover, the general concept of phage display is well-known to the person skilled in the art and described herein above.
In this embodiment mapping of epitopes on (poly)peptides in (poly)peptide libraries of the invention by phage display is envisioned (cf. Example 1). Accordingly, the vector used for cloning the coding elements is a fusion vector allowing the expression of the coding element fused to a phage. Since the vector in contrast to methods of the state of the art is a phagemid, the method as claimed has several advantages. The (poly)peptide libraries, e.g., can be expanded without loss of diversity since the fusion protein is only expressed when induced by addition of arabinose to the medium (cf. Example 1). The libraries can be maintained as bacterial plasmids and expressed *in situ* or incorporated into phage when necessary by the addition of helper phage to the medium.

Moreover, the invention also relates to a method for generating protein variants comprising the steps of:
(a) preparing a nucleic acid molecule as defined above; wherein at least one coding element comprises a variant sequence in comparison to the corresponding wild-type sequence of a target protein; and wherein the coding elements when arranged as a continuous sequence encode the entire target protein variant;
(b) performing an amplification step with the nucleic acid molecule obtained in step (a) and primers hybridizing to the sequence of said linking elements so that the sequences comprising the coding elements (A) are specifically amplified;
(c) combining the coding elements obtained in step (c) and performing a primerless polymerase chain reaction (PCR);
(d) performing a PCR with the amplicons obtained in step (d) and a primer pair that results only in amplification of amplicons encoding the entire target protein variant;
(e) cloning the amplicons encoding the entire target protein variant obtained in step (e) into vectors;
(f) transforming the vectors obtained in step (f) into host cells and culturing said host cells under conditions suitable to express the target sequences encoding the target protein variants; and
(g) identifying host cells that express the target protein variants.

The terms "variant sequence", "amplification", "hybridizing" as well as "vectors" and cell culture conditions have been described herein above.

The term "identifying" as used in step (g) of this embodiment describes the process of determining which host cells express variant (poly)peptides. This may, as exemplarily shown in Example 2 (cf. example section infra), be achieved by using a monoclonal antibody recognizing an epitope shared by all variant (poly)peptides. The clones expressing the variant (poly)peptides may essentially be visualized by any form of "immunological screening" as described above. Subsequently, the coding element of the clones expressing the variant (poly)peptide are sequenced in order to correlate the specific variant (poly)peptide to the clone expressing it. In this way, a library of variant (poly)peptides may be established which is also claimed as part of this invention.

The term "primerless PCR" refers to a method of amplifying nucleic acid fragments without the addition of primers, i.e. the nucleic acid fragments act as primers themselves. Primerless PCR is used to build up a complete gene from a population of overlapping gene fragments usually generated by random cutting of the DNA of interest with DNase I. In order to mix related DNAs from different genes the two Dnase I digested samples are mixed and subjected to a limited number of rounds of PCR amplification in the absence of primers. The different fragments anneal to each other and are extended by the action of the thermally resistant DNA polymerase. This process results in the assembly of whole genes in which the two homologous genes have been randomly mixed. Finally primers are added and the population of gene variants is amplified by normal PCR in order to obtain a population of DNA molecules that can cloned (Suenanga H, Goto M, Furukawa K. DNA shuffling in Evolutionary Methods in Biotechnology Brakmann S, Schwienhorst A (Eds) Wiley-VCH (2006)).

This embodiment may be used as a rational approach to designing and generating a library of variant (poly)peptide sequences derived from one starting sequence. As evidenced in Example 2, by this approach variant (poly)peptides may subsequently bye subjected to a mapping assay in order to identify an epitope in the variants bound by a single antibody. An epitope identified accordingly may, e.g., in the case of several subtypes of an infectious agent, be used in the development of a vaccine as a means of prophylactic treatment as well as neutralizing antibodies to be administered as treatment of an acute infection with one or more of said subtypes of an infectious agent.

In a variation of the method of the above embodiment the nucleic acid molecule of step (a) is cloned into a vector, subsequently a host cell is transformed with said vector and propagated and, finally, the vector is isolated from said host cell to be modulated and processed as described herein above to yield single coding elements and further processed as in steps (c) to (g). This variation may be used when there are only few variant sequences and constant sequences to be amplified in cell culture and the resulting libraries are of low complexity.

In a preferred embodiment of the method of the invention, coding elements comprising variant sequences and coding elements comprising wild-type sequences are comprised by separate nucleic acid molecules.

When variant and wild-type coding sequences are part of different nucleic acid molecules of the invention one can establish two different pools of coding elements, i.e. one comprising only wild-type coding elements and the other comprising only variant sequences. The pool of the variant coding elements may further be subdivided in sub-pools. Instead of having to generate all variants, the partition of wild-type and variant coding elements in separate (sub-)pools provides the advantage of not having to generate all (poly)peptide variants but only selected (poly)peptide variants.

The invention also relates to a nucleic acid molecule as defined in herein above.

Also, the invention relates to a vector comprising the nucleic acid molecule of the invention.

Further, the invention relates to a cell comprising the nucleic acid molecule of the invention or the vector of the invention. Vectors, cloning methods and suitable host cells have been described herein previously and are, moreover, well known to the person skilled in the art.

Finally, the invention relates to a kit comprising one or more items of the group selected from a nucleic acid molecule according to the invention, a vector according to the invention, a cell according to the invention, an RNA library or (poly)peptide library according to the invention and, optionally, instructions for use.

The one or more components of the kit may be packaged in one or more containers such as one or more vials. The vials may, in addition to the components, comprise preservatives or buffers for storage, media for maintenance and storage, e.g. in the case of cells, cell media, DMEM, MEM, HBSS, PBS, HEPES, hygromycin, puromycin, Penicillin-Streptomycin solution, gentamicin inter alia. Advantageously, the kit further comprises instructions for use of the components allowing the skilled person to conveniently work, e.g., various embodiments of the invention.

The figures show:
**Figure 1****: Blast alignment from the E7 proteins from human papillomavirus types 16 and 18**
   Alignment of the E7 proteins from type 16 and type 18 HPV showing the (poly)peptides from the type16 protein that were isolated from the panning experiment using serum from animals immunized with type 18 E7 protein. The red residues are identical in the two proteins and indicate the basis for the occurrence cross-reactive antibodies.
**Figure 2****: Plasmid vector pLM-araPgVIII(4)**
   Plasmid vector pLM-araPgVlll(4) used for expression of (poly)peptide libraries. The gVIII product is only expressed when an in-frame insert is introduced. Expression is induced by addition of arabinose to the medium.

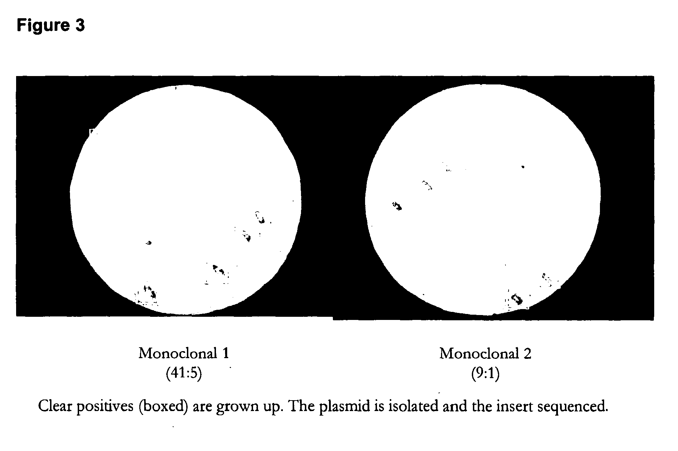

**Figure 3****: Colony blotting of clones expressing gVIII fusion products**
   Colony blotting of clones expressing gVIII fusion products carrying (poly)peptides from the E7 protein of human papillomavirus type 16.
   The colonies were screened with two monoclonal antibodies raised against recombinant E7 protein. A goat anti-mouse IgG conjugated to horseradish peroxidase was used to visualize positive clones using hydrogen peroxide as substrate and o-chloronaphthol as chromogen. White boxes indicate clear positives.

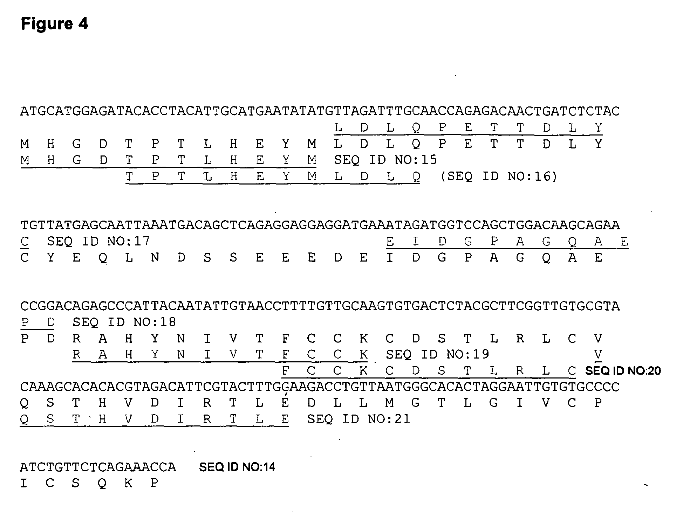

**Figure 4****: Identification of HPV sequences bound by antibodies**
   Red sequences are those in clones recognized by antibody 41:5. The blue sequence was in all the clones recognized by antibody 9:1. The green sequences are from negative clones that were adjacent to positive clones on the template.
**Figure 5****:**
   The inserts from clones recovered after a single round of panning. The binding appears to be highly specific since clones representing a single region are heavily over-represented.
**Figure 6****:**
   The results show clearly that the specificity is highest when screening for responses to the homologous protein. It is also clear that there is a dominant epitope locate at the amino terminus of the protein since 80% of all the sequences contained (poly)peptides from this region. Because of the high failure rate of the sequencing reactions with the type 18 serum. Further sequences are required to determine whether the responses observed are specific.
**Figure 7****:**
   The three toxin B subunits showing the constant (colored) regions and the variable regions (black). The signal (poly)peptide is derived from LTB but the coding region depicted is CTB. The bases above or below the DNA sequence of CTB are mutations required to alter the amino acid at the corresponding position in LTB or CitTB respectively. The native residue at the N -terminus of CTB is threonine, but in our recombinants is this mutated to alanine (shown with an asterisk). The amino acid sequences are shown in red. Residues that differ at any position relative to the other two sequences are shown in black. The * below the sequences inducate positions at which a single base change is sufficient to cause the desired amino acid substitution whereas ! indicates positions at which the at least two mutations are required.
**Figure 8****:**
   The colored constant regions are released by digestion with Haell and Nael followed by blunt-end repair with either T4 DNA polymerase or S1 nuclease.
**Figure 9****: Exemplary pattern of overlapping fragments of a given sequence**
   A-D depict different modes of dividing a (poly)peptide into fragments for use, e.g., a scanning library for the detection of antibody epitopes.
**Figure 10****:**
   Synthesis of variable regions as libraries showing single base-changes and the number of variants each synthesis will give rise to.
**Figure 11****:**
   Nucleic acid molecule synthesis for maximum complexity of a toxin B subunit variant library.

The examples illustrate the invention:

### Example 1: The mapping of E7 monoclonal and polyclonal antibodies using a (poly)peptide library

An overlapping (poly)peptide library was generated for the E7 protein of type 16 human papilloma virus.
The protein has the sequence:
   MHGDTPTLHEYMLDLQPETTDLYCYEQLNDSSEEEDEIDGPAGQAEPDRAHYNIV TFCCKCDSTLRLCVQSTHVDI RTLEDLLMGTLGIVCPICSQKP
   (98 amino acids; SEQ ID NO: 1)

The following overlapping peptides were proposed (66% overlap):

**TABLE 1**

| Overlap: from residue | Peptide sequences. |
|---|---|
| #1 SEQ ID NO: 2 | NH2-MHGDTPTLHEYM LDLQPETTDLYC YEQLNDSSEEED EIDGPAGQAEPD RAHYNIVTFCCK CDSTLRLCVQST HVDIRTLEDLLM GTLGIVCPICSQ-COOH |
| #5 SEQ ID NO: 3 | NH2-TPTLHEYMLDLQ PETTDLYCYEQL NDSSEEEDEIDG PAGQAEPDRAHY NIVTFCCKCDST LRLCVQSTHVDI RTLEDLLMGTLG LGIVCPICSQKP-COOH |
| | |
| #9 SEQ ID NO: 4 | NH2-HEYMLDLQPETT DLYCYEQLNDSS EEEDEIDGPAGQ AEPDRAHYNIVT FCCKCDSTLRLC VQSTHVDIRTLE DLLMGTLGIVCP-COOH |

A total of 23 peptides.

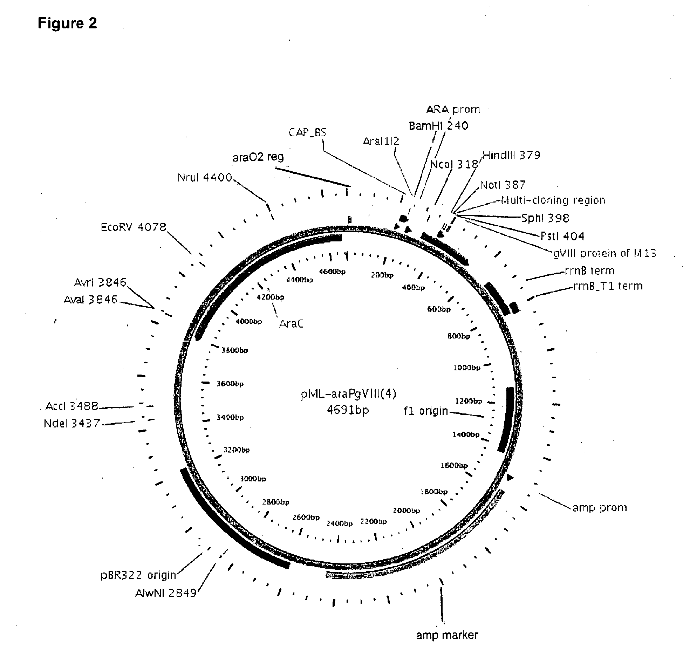

The oligonucleotides were synthesized in order to generate coding elements encoding the above peptides such that they could be inserted in the correct reading-frame into an appropriate expression vector. In this case the expression vector was pML-araPgVIII(4) the DNA structure of which is shown in Fig. 2.

The coding elements are flanked by HindIII and Pstl sites and additional bases that adjust the reading frame relative to the gVIII protein of M13 to which they will be fused. The orientation of the coding elements is alternated in order to minimize the number of bases that need to be synthesized. The order of the coding elements is designed to minimize structures that complicate synthesis such as long direct or inverted repeats.

The synthesized sequences are shown below. The underlined regions are the linkers carrying the restriction endonuclease recognition sites used in subsequent cloning procedures. The colours refer to the peptide sequences shown in Table 1.
**Sequence #1 SEQ ID NO:5:** 
5′-AAGCTTTGCCATGCATGGAGATACACCTACATTGCATGAATATATGCCTGCAGGTGGTTTCT
GAGAACAGATGGGGCACACAATTCCTAGGGCAAAGCTTTGCCTTAGATTTGCAACCAGAGACAAC
TGATCTCTACTGTCCTGCAGGTCCTAGTGTGCCCATTAACAGGTCTTCCAAAGTACGGGCAAAGC
TTTGCCTATGAGCAATTAAATGACAGCTCAGAGGAGGAGGATCCTGCAGGCGTAGAGTCACACTT
GCAACAAAAGGTTACAATATTGGCAAAGCTTTGCCGAAATAGATGGTCCAGCTGGACAAGCAGAA
CCGGACCCTGCAGGAATGTCTACGTGTGTGCTTTGTACGCACAACCGAAGGGCAAAGCTT-3′
**Sequence #2 SEQ ID NO:6:** 
5'-AAGCTTTGCCAGAGCCCATTACAATATTGTAACCTTTTGTTGCAAGCCTGCAGGACCATCTA
TTTCATCCTCCTCCTCTGAGCTGTCATTGGCAAAGCTTTGCCTGTGACTCTACGCTTCGGTTGTG
CGTACAAAGCACACCTGCAGGGTAATGGGCTCTGTCCGGTTCTGCTTGTCCAGCTGGGGCAAAGC
TTTGCCCACGTAGACATTCGTACTTTGGAAGACCTGTTAATGCCTGCAGGTAATTGCTCATAACA
GTAGAGATCAGTTGTCTCTGGGGCAAAGCTTTGCCGGCACACTAGGAATTGTGTGCCCCATCTGT
TCTCAGCCTGCAGGTTGCAAATCTAACATATATTCATGCAATGTAGGTGTGGCAAAGCTT-3'
**Sequence #3 SEQ ID NO:7:**

5′-AAGCTTTGCCCATGAATATATGTTAGATTTGCAACCAGAGACAACTCCTGCAGGGGGCACA
CAATTCCTAGTGTGCCCATTAACAGGTCGGCAAAGCTTTGCCGATCTCTACTGTTATGAGCAATT
AAATGACAGCTCACCTGCAGGTTCCAAAGTACGAATGTCTACGTGTGCTTTGTACGGCAAAGC
TTTGCCGAGGAGGAGGATGAAATAGATGGTCCAGCTGGACAACCTGCAGGGCACAACCGAAGCGT
AGAGTCACACTTGCAACAAAAGGCAAAGCTTTGCCGCAGAACCGGACAGAGCCCATTACAATATT
GTAACCCCTGCAG-3′

The synthesized nucleic acid molecules were cloned as blunt-ended fragments into the EcoRV site of the standard vector pKS1 a derivative of pUC19. The syntheses were then checked by sequencing the inserts in each of the recombinant plasmids (pS382-1 carrying sequence #1, pS382-2 carrying sequence #2 and pS382-3 carrying sequence #3).

The nucleic acid molecules were transformed into the standard cloning recipient strain XL1 Blue. The resulting strains were maintained as frozen stocks at -70 °C. Plasmid DNA was prepared from each of the strains (Fermentas Jetprep kit). 1 µg of each plasmid was digested to completion with PstI and HindIII. Following digestion the coding elements were treated with alkaline phosphatase in order to dephosphorylate the ends of the coding elements.

A similar quantity of pML-araPgVlll(4) was also digested to completion with Pstl and HindIII.

Both DNA samples were cleaned up (Omega DNA clean-up kit, protocol for small fragment recovery).

The digested and dephosphorylated DNA samples containing the coding elements derived from the nucleic acid molecules were mixed individually with the digested expression vector. An additional sample contained all three digested plasmids in order to obtain a single library that contained all the (poly)peptides that could be derived from the three synthetic nucleic acid molecules. The vector was added in all reactions so that the coding elements to be cloned were in a molar excess of approximately 10:1. The DNA mixtures were then ligated with T4 DNA ligase in the buffer supplied by the manufacturer. Reactions were left at room temperature overnight.

Ligated DNA was used to transform commercially obtained electro-competent DH12S cells (Invitrogen). Electroporated cells were grown up in SOC medium for approximately 1 h and then transferred to 25 ml of fresh LB broth containing 100 µg/ ml ampicillin. 10 and 20 µl aliquots were plated out onto LB agar containing 100 µg/ ml in order to determine the transformation frequency.

Once the recombinant library had grown up 2 ml aliquots were supplemented with 1 ml 50% glycerol and maintained as frozen stocks at -70 °C. A further 5 ml of the culture was used to prepare plasmid DNA for analysis.

DNA samples were digested with Eam11051 (AhdI) and NotI and the resulting fragments were resolved on 1 % agarose gel run in Tris Borate EDTA (TBE) buffer. Bands were visualized by post run staining with ethidium bromide.

The digests showed that although the background of pKS plasmid had been considerably reduced by the dephosphorylation, it had not been removed completely. However, they also showed that there was an extremely low background of background expression vector lacking insert (the vector contains a NotI site that is removed if there is an insertion between the HindIII and PstI sites).

Two approaches were taken to remove the contaminating pKS vector. The first was to digest the DNA samples with Xhol and transform it again into the electro-competent DH12S cells. This succeeded in further reducing background, but to negligible levels in only one of the four samples (containing clones from construct #3).

In order to definitively remove the background, 2 mg DNA was digested with Eam1105I and NotI. The digested DNA was resolved on a 1% agarose gel and stained with ethidium bromide as described previously. The band representing the expression vector DNA carrying insert was cut out from the gel. The gel fragment was dissolved and the DNA extracted using a gel extraction kit (Qiagen). The extracted DNA ligated using T4 DNA ligase and used to transform electro-competent TOP10F' cells. Transformation of the commercially available cells was completely inhibited by residues from the band extraction that proved impossible to remove. This resulted in a far lower transformation frequency but, because of the small total number of (poly)peptides, sufficient transformants were generated to ensure that the entire library was represented.

Future modification of the cloning vectors will result in considerably reduced problems with background. For example, the synthesized DNA can be cloned into a vector with a different selection marker from the expression vector so that counter selection is made easier. Another alternative is to use a cloning vector that does not carry the f1 origin of replication so that the phagemid expression vector can be separated from the background by superinfection with a helper phage and creating a phage stock. This can be used to transfer the phagemids into a suitable bacterial host to be maintained as a plasmid library.

### Direct mapping of epitopes recognized by E7 type 16-specific monoclonal antibodies.

135 colonies from the library covering the entire mixture of E7 (poly)peptides were plated out onto a grid on duplicate 13.8 cm plates containing LB agar supplemented with 100 mg/ml ampicillin. When the colonies had grown up a sterile nitrocellulose filter that had been moistened was placed onto the plate and left for approximately 20 seconds. It was then carefully removed and placed with the colony side upwards on a fresh 13.8 cm LB plate supplemented with ampicillin as before, but also with arabinose at a concentration of 0.1 %.
The process was repeated, lifting colonies a second time from the same plate and placing the resulting nitrocellulose filter on a second fresh 13.8 cm LB plate supplemented with ampicillin and arabinose. The duplicate plate was used as a template for recovery of identified clones and was stored at 4 °C.
The plates containing nitrocellulose filters were incubated at 37 °C overnight on order to allow the colonies to grow under inducing conditions. The colonies were then lysed *in situ* and subjected to immunological screening essentially according to the methods described by Sambrook and Russell, 2001. Each filter was used for detection of clones expressing epitopes recognized by a different monoclonal antibody.
On one filter six colonies gave clear signals and on the second filter seven colonies gave clear signals (cf. Fig. 2). These colonies were identified on the template streaked onto fresh LB plates supplemented with ampicillin and plasmid DNA was prepared from each of them. In addition, six negative colonies that were adjacent to the positive clones on one of the plates were taken and treated in the same manner. The plasmids were sequenced using a primer that allowed the sequence of the insert to be determined in each case. The results are shown in Fig. 4.
The monoclonal antibody 9:1 recognized a single clone with sequence EIDGPAGQAEPD (SEQ ID NO: 8). From previous mapping studies it was expected that two clones in the library should have been recognized by the monoclonal antibody, it may be that some of the clones are not represented in the array that was used.
The monoclonal antibody 41:5 recognized two clones with the sequences MHGDTPTLHEYM (SEQ ID NO: 9) and TPTLHEYMLDLQ (SEQ ID NO: 10). In this case the common sequence shared by both clones encodes the sequence TPTLHEYM (SEQ ID NO:11). This is the limit of resolution that can be achieved with the overlap present in the (poly)peptide array used. A third overlapping clone was not detected.

### Analysis of immune responses to the E7 protein in crude serum from immunized and non-immunized mice.

In the case of serum samples from immunized mice the colony blotting method did not function. The mice were immunized with recombinant E7 protein derived from *E. coli*. This meant that the background due to antibodies against contaminating host proteins in the E7 preparations was too high.

An alternative panning approach was taken. The clone library carrying all twenty three (poly)peptides were used to generate a phage displayed array. A sample of the clone library was cultured at 37 °C with shaking (180 rpm) overnight in LB broth supplemented with ampicillin (100 µg/ml). 20 µl of this culture was used to inoculate 2 ml TB medium also supplemented with 100 µg/ml ampicillin. At the same time 10 µl of a commercially available stock of the helper phage M13KO7 was added (Invitrogen, ∼10¹¹ pfu/ml). The culture was grown at 37 °C with shaking (180 rpm) for 2h. Kanamycin was then added to the culture to a final concentration of 50 µg/ml and at the same time, arabinose was added to a final concentration of 0.1 %. These additions ensured that only cells infected with the phage could grow and that those cells were expressing the recombinant gVIII fusion proteins.
The culture was then incubated under the same conditions for a further 16h. The cells were then removed first by centrifugation and subsequently by passage of the culture supernatant through a 0.2 micron filter.
The number of phages carrying the pML-araPgVIII(4) derived phagemids in the resulting suspension was then determined. The suspension was serially diluted in TB medium to a final dilution of 10⁻⁶. 10 µl of the diluted phage suspension was added to 200 µl of a fresh culture of *E. coli* strain TOP10F'. The culture was incubated for 90 minutes at 37° C with shaking (180 rpm) and the entire culture was then spread onto a fresh LB agar plate containing 100 µg/ml ampicillin and incubated at 37° C overnight. The total number transformants representing the number of phagemid carrying particles was then calculated. The number of particles obtained was 3.6 x 10⁹/ ml of suspension.

Wells in a 96 well Greiner bio plate were coated overnight at 4°C with 150 µl of 1/100 dilutions of total serum from BalbC mice that had been immunized E7 derived either from type 16 or typ18 human papillomavirus. Dilutions were made in 50 mM Sodium carbonate buffer pH 8.7. A similarly diluted control serum was taken from a naive mouse.
Coated plates were washed and blocked with 0.1 M NaHCO₃ buffer pH 8.7 containing 5 mg/ml bovine serum albumin (BSA) for 1 h at 4° C with gentle agitation. The wells were then washed six times with 50 mM Tris-HCl pH7.5 containing 150 mM NaCl, 0.1% Tween 20 (TBST buffer). 100 µl undiluted phage suspension (3.6 x 10⁸ phages) were added to each of the coated plates and left for 1 h at room temperature with gentle rocking. The phage suspension was removed and unbound phages were removed by washing eight times with TBST buffer.
Bound phages were eluted by addition of 100 µl 0.2 M glycine-HCl pH 2.2, 1 mg/ml BSA and incubation at room temperature for 12 m with gentle rocking. Elution budfer was then removed from the wells and neutralized by addition of 15 µl 1 M Tris pH 9.1.

The eluted phages were then used to infect a culture of TOP10F' in order to calculate the total number of eluted phages as already described. The results for each of the serum samples are shown below.

**Table 2**

| Immunization | Recovered phagemids^{*} |
|---|---|
| HPV E7 type 16 | 2.8 x 10 ⁴ |
| HPV E7 type 18 | 6.2 x 10³ |
| Blank, non-immunized | 4 x 10² |

| | |
|---|---|
| *Total number of phagemids recovered from a single round of panning using crude serum from mice immunized with different proteins. Only the phagemid carrying particles were counted. Numbers of true phages were not counted, but packaging of helper phage is usually at least one order of magnitude lower than for the phagemids in this sytem. The highest number was expected from the mice immunized with the homologous antigen (E7 from type 16 HPV). A degree of cross-reactivity is also expected from the mice immunized with type 18 E7 protein, with the lowest titre, indicating nonspecific background expected from the non-immunized mice that have never see the antigen. | |

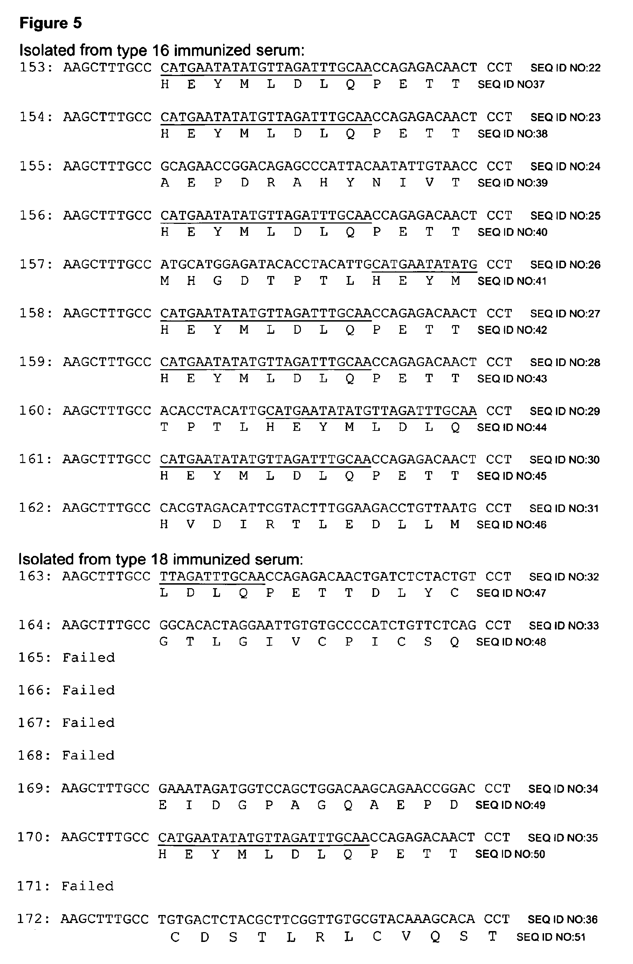

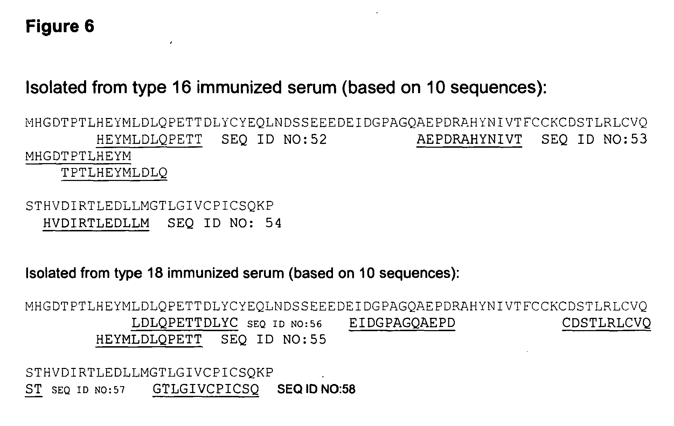

Twenty clones from each of the eluted samples were isolated and plasmid prepared. The insert encoding the E7 (poly)peptide was sequenced in each of them (cf. Fig. 5). The results show clearly that the dominant epitope for the antigen in the form in which it was immunized is situated at the amino terminus of the protein (cf. Fig. 6). The minimal sequence was determined to be HEYM since all the overlapping (poly)peptides from that region of the protein were detected.

The reactivity with the serum from the type18 E7 immunized mice was designed to show cross-reactive epitopes.

The E7 protein from human papillomavirus type 16 has the sequence:
MHGDTPTLHEYMLDLQPETTDLYCYEQLNDSSEEEDEIDGPAGQAEPDRAHYNIV TFCCKCDSTLRLCVQSTHVDIRTLEDLLMGTLGIVCPICSQKP
(98 amino acids; SEQ ID NO: 12)

The E7 protein from human papillomavirus type 18 has the sequence:
MHGPKATLQDIVLHLEPQNEIPVDLLCHEQLSDSEEENDEIDGVNHQHLPARRAEP QRHTMLCMCCKCEARI ELWESSADDLRAFQQLFLNTLSFVCPWCASQQ
(105 amino acids; SEQ ID NO: 13)

### Example 2: Generation of chimera chimera proteins based on three naturally occurring variants of the cholera toxin B subunit in order to screen for altered receptor binding properties

The cholera toxin B subunit (CTB) is probably the best understood and most widely studied of a class of proteins that constitute the B subunit of AB₅ toxins. In these toxins the A subunit is the toxic moiety responsible for the toxic activity of the holotoxin, whereas the B subunits form a homopentamer with which the A subunit is non-covalently associated. The B subunit pentamers are responsible for binding of the toxin to target cells via specific receptors. In the case of CTB, the receptor has been identified as the ganglioside GM1. The binding is extremely avid and highly specific. Two other closely related toxin B subunits have been identified. These are the B subunit of the heat-labile toxin of enterotoxigenic *Escherichia coli* (LTB) which is structurally and functionally closely related to cholera toxin and shares 83% identity at the protein level, and the uncharacterized toxin from *Citrobacter freundii* the subunit (CitTB) of which shares about 73% identity with the other two B subunits. Despite their structural similarities which have been confirmed by X-ray crystallography, and their similar affinity for GM1 ganglioside, the different B subunits do exhibit different receptor binding properties that have been well characterized. Furthermore chimeras generated between CTB and LTB not only showed receptor binding characteristics intermediate between the two parental subunits, but also acquired novel binding characteristics that were absent from both parental B subunits. One observed gain of function was the ability to bind to blood group A and B antigens via a novel binding site situated at a position distant from the GM1 binding site. From analysis of the CitTB structure it was predicted that this molecule should also bind to blood group A and B antigens and when the molecule was expressed from a synthetic gene this was indeed found to be the case. It is clear from previous studies that the observed differences in primary structure of the different proteins lead to differences in the properties of the different subunits with respect to their receptor binding properties.
For several reasons the B subunit of LTB and CTB are of interest. They are for example extremely stable molecules that spontaneously assemble into receptor-binding pentamers. In humans they are non-toxic and yet immunogenic in their own right; giving rise to antibodies that will neutralize the holotoxin. This has led to the inclusion of CTB into a registered oral vaccine against cholera. It is possible to use the molecules as carriers for the administration of foreign antigens to the immune system in order to elicit both positive immunity and to induce immunological tolerance. Furthermore, it is possible to genetically manipulate the molecules in order to incorporate (poly)peptide antigens into their structure. It is unclear whether the differing receptor binding properties affect the ability of the different B subunits to act as carriers or whether they have a profound affect on the immunological properties of the molecules. However, important as these questions are, it is also of considerable interest that these molecules have potentially multiple receptor binding sites with affinities for different sugar structures and that systematically produced chimeras can gain affinities for ligands not recognized by the parental structures. A strategy for introducing variations into the basic toxin B subunit molecule has therefore been devised in which a library is constructed containing mutatants that vary only at the positions of the 103 amino acid proteins where there are naturally occurring variations. Furthermore, the mutations are limited to those that allow the substitution of an amino acid present at the corresponding position of one of the three variant starting molecules (CTB,LTB and CitTB).

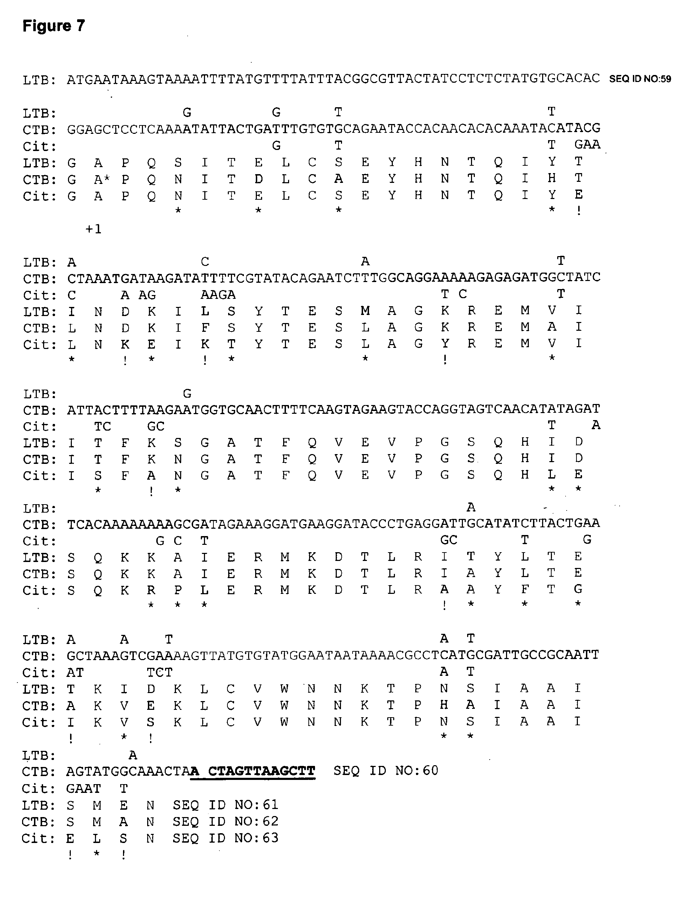

Firstly, all the common amino acids shared by all three molecules were standardized so that the codon representing them was the same in each molecule.
Secondly the codons at the positions at which variations occur were altered so that substitutions could be made by a single base mutation. In some cases this was not possible and substitutions required more than one mutation in a single codon. The result of these manipulations of the sequence is summarized in Fig. 7.

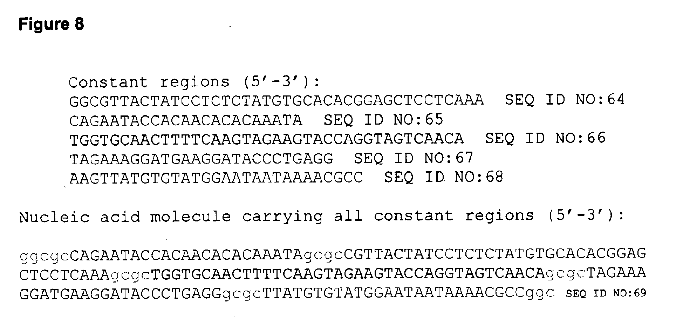

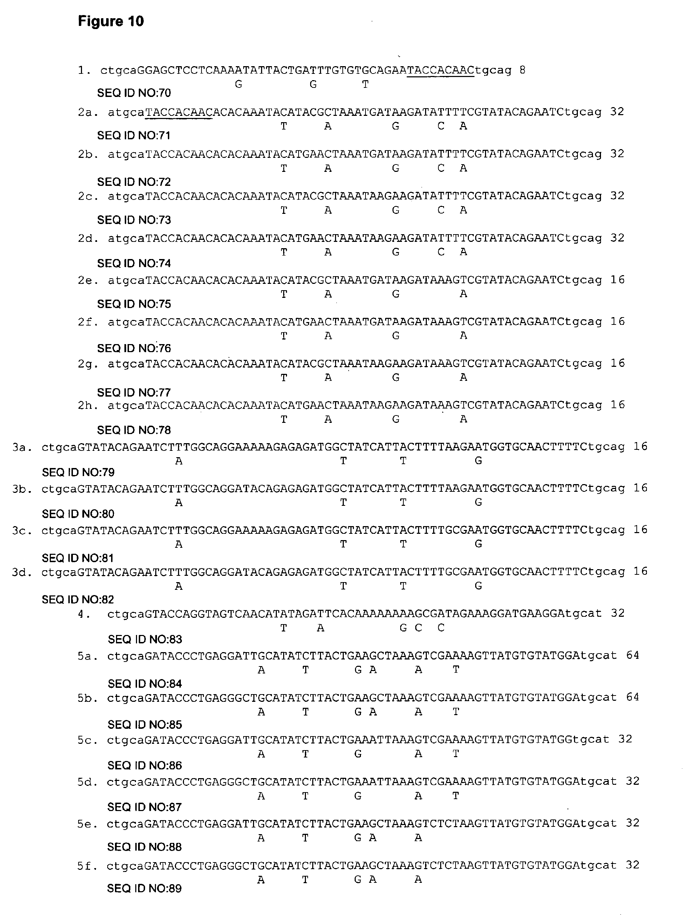

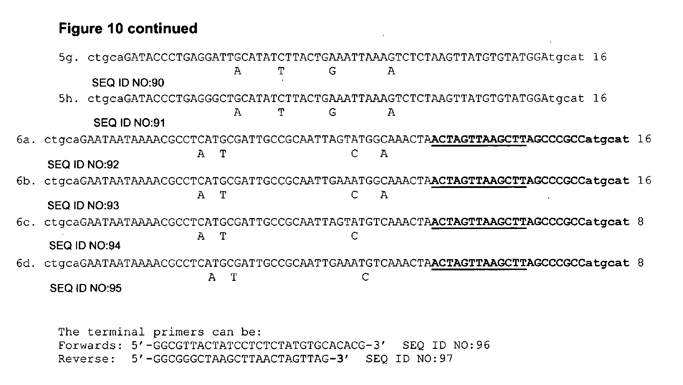

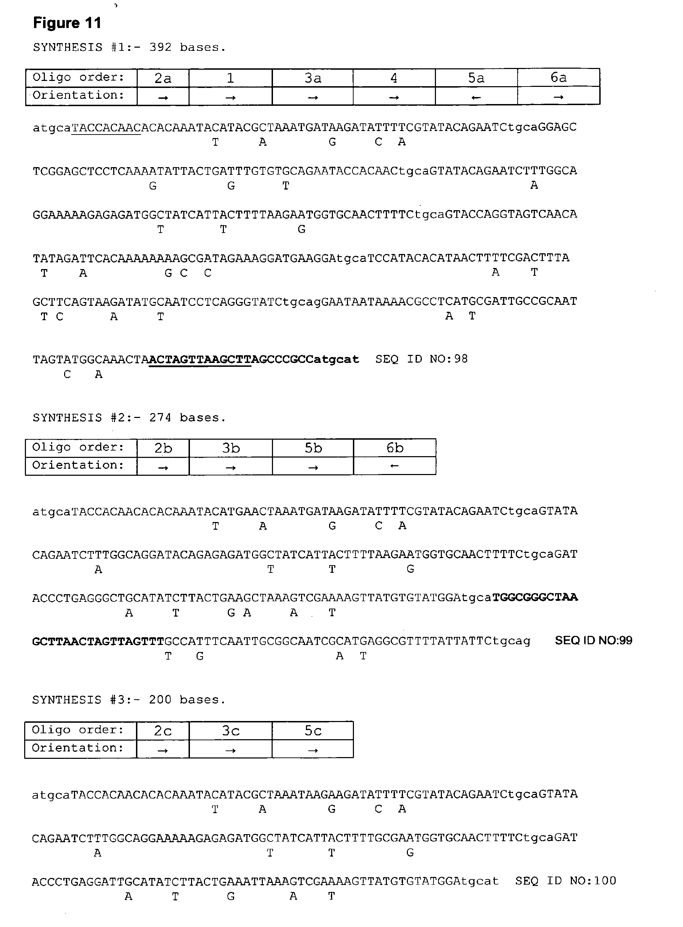

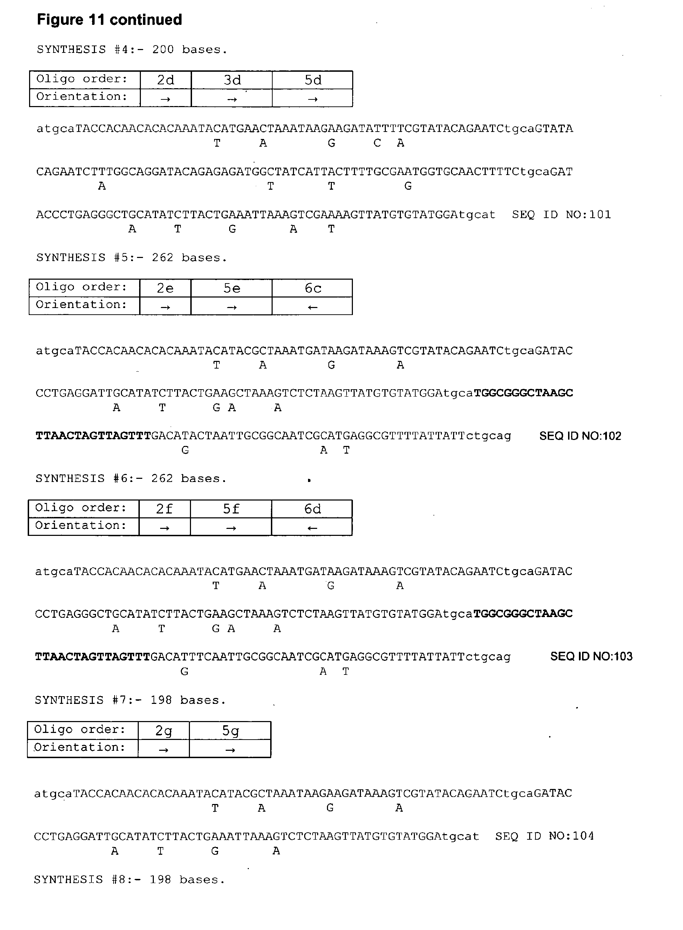

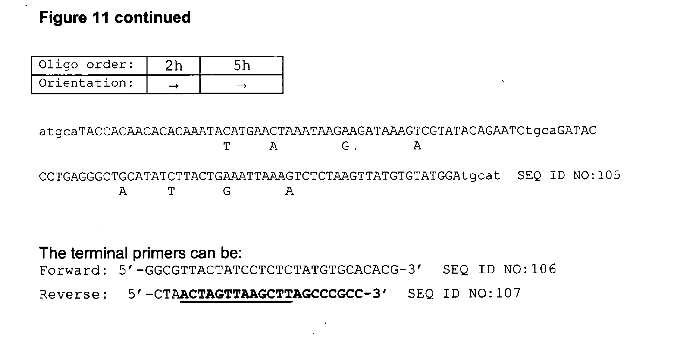

Two sets of nucleic acid molecules could then be synthesized. One nucleic acid molecule containing the shared constant regions of the molecules (cf. Fig. 8), the second a nucleic acid molecule carrying variable regions each with wobbles at specific sites giving codon changes that would result in the desired substitutions and no others. The coding elements needed to achieve this are shown in Fig. 10. An outline of the different coding elements that must be constructed to give maximum complexity to the library is shown in Fig. 11. The level of complexity obtained in any library can be controlled by manipulating the variable coding elements one adds to subsequent amplification reactions. Additional coding elements can be made that have variations in only some regions leaving the others with an invariable default sequence that corresponds to a chosen starting molecule (in this case CTB, LTB or CitTB).
In a preliminary experiment the nucleic acid molecule containing the constant regions and a mixture of three different nucleic acid molecules containing variants were used. The nucleic acid molecule containing the variants must be synthesized, cloned and transformed such that the frequency of transformation is sufficient to cover the entire number of variations with a high degree of degeneracy. Alternatively, the number of cloning steps can be reduced by using a PCR product derived from the synthesized nucleic acid molecule directly. In the described experiment three clones were taken from the library at random. They were sequenced and it was confirmed they each contained different variations.
In this setup the different oligonucleotides were linked by six-cutting restriction endonucleases that gave rise to either blunt ends (Nael in the constant region nucleic acid molecule) or 3' extensions (Haell in the constant region nucleic acid molecule and PstI and NsiI in the variable region nucleic acid molecule).
The individual plasmids (or PCR fragments) are digested to completion with the appropriate enzymes in separate reactions and then treated with T4 DNA polymerase to obtain blunt ended fragments in which the 3' extensions have been removed. The digested coding elements are then subjected to primerless PCR followed by standard PCR with primers that amplify all the resulting variant toxin B subunit molecules. The DNA is then digested with SacI and HindIII in order to clone it into an expression vector designed for over-expression of CTB.
The ligated DNA is transformed into a suitable *E. coli* host strain. Transformants are then screened for expression of pentameric B subunits using a monoclonal antibody that recognizes all the different structures.
A random selection of the expressing clones are then taken for sequencing.
The strategy effectively involves the synthesis of mini-libraries with limited complexity and mixing them randomly in a PCR-based shuffling procedure to achieve maximum complexity of the reassembled genes.
The approach has the advantage that the level of complexity achieved can be varied by selection of the different sequence sets one wishes to add to the amplifications.

## Claims

1. A method for generating an RNA library or a (poly)peptide library comprising the steps of:
(a) providing one or more nucleic acid molecules each comprising
i) two or more coding elements (A) each giving rise to an RNA molecule upon transcription and/or a (poly)peptide upon transcription and translation; and
ii) linking elements (B)
arranged according to the general formula of B(AB)₂₊ₙ, wherein said linking elements comprise one or more sequence motifs not found in said two or more coding elements allowing specific disruption of the linking elements (B);
(b) cloning the nucleic acid molecule of step (a) into a vector;
(c) transforming a host cell with the vector obtained in step (b) and propagating said transformed cell;
(d) preparing vector DNA from the transformed and propagated cells of step (c);
(e) (i) disrupting the vector DNA obtained in step (d) with one or more agents recognizing said one or more sequence motifs of the linking elements or (ii) performing an amplification step with the vector DNA obtained in step (d) and primers hybridizing to the sequence of said linking elements so that the sequences comprising the coding elements (A) are specifically amplified;
(f) cloning the resulting coding elements (A) of step (e) into vectors;
(g) transforming the vectors obtained in step (f) into host cells and establishing clonal colonies; and
(h) culturing said clonal colonies under conditions suitable to express the coding elements.

2. The method of claim 1, wherein the sequence of the coding elements (A) is different.

3. The method of claim 1 or 2, wherein sequence of the linking elements (B) is the same.

4. The method of any one of claims 1 to 3, wherein the disruption in step (e) generates coding elements devoid of sequence fragments of linking elements.

5. The method of any one of claims 1 to 4, further comprising after step (h) the additional step (i) of isolating the RNA molecule and/or (poly)peptide encoded by the coding element of each clonal colony.

6. The method of any one of claims 1 to 5, wherein the two or more coding elements when arranged as a continuous sequence comprise the entire or partial coding sequence of one or more genes in the native reading frame.

7. The method of claim 6, wherein the coding elements when arranged as a continuous sequence comprise overlapping sequences.

8. The method of any one of claims 1 to 7, wherein one or more coding elements comprise a sequence variant of a sequence.

9. The method of any one of claims 1 to 8, wherein the nucleic acid molecule is synthetically produced.

10. The method of any one of claims 1 to 8, wherein the vector in step (f) is a fusion protein vector allowing the expression of a (poly)peptide encoded by a coding element fused to a phage protein for phage display.

11. An RNA library or a (poly)peptide library obtainable or obtained according to the method of any one of claims 1 to 10.

12. A method for identifying a (poly)peptide epitope recognized by an antibody or a (poly)peptide-binding compound comprising the steps of:
(a) preparing a (poly)peptide library according to the method of any one of claims 1 to 4 and 6 to 9;
(b) subjecting said clonal colonies of the (poly)peptide library to immunological screening with the antibody of interest to identify clonal colonies expressing a (poly)peptide that is bound by said antibody or said (poly)peptide-binding compound of interest; and optionally
(c) based on the result obtained in step (b) sequencing the coding elements of the vectors of the clonal colonies to identify the (poly)peptide epitope recognized by said antibody or said (poly)peptide-binding compound.

13. A method for identifying a (poly)peptide epitope recognized by antibodies in serum comprising the steps of:
(a) preparing a (poly)peptide library according to the method of claim 10;
(b) infecting the clonal colonies with a helper phage and obtaining phages carrying the fusion protein vector for each clonal colony;
(c) contacting the phages obtained in step (b) with the serum;
(d) determining binding of phages to antibodies in said serum; and optionally
(e) based on the result of step (d) sequencing the coding element of the vector of the bound phages to identify the (poly)peptide epitopes recognized by antibodies in said serum.

14. A method for generating protein variants comprising the steps of:
(a) preparing a nucleic acid molecule as defined in any one of claims 1 to 4 and 6 to 9; wherein at least one coding element (A) comprises a variant sequence in comparison to the corresponding wild-type sequence of a target protein; and wherein the coding elements when arranged as a continuous sequence encode the entire target protein variant;
(b) performing an amplification step with the nucleic acid molecule obtained in step (a) and primers hybridizing to the sequence of said linking elements so that the sequences comprising the coding elements (A) are specifically amplified;
(c) combining the coding elements obtained in step (c) and performing a primerless polymerase chain reaction (PCR);
(d) performing a PCR with the amplicons obtained in step (d) and a primer pair that results only in amplification of amplicons encoding the entire target protein variant;
(e) cloning the amplicons encoding the entire target protein variant obtained in step (e) into vectors;
(f) transforming the vectors obtained in step (f) into host cells and culturing said host cells under conditions suitable to express the target sequences encoding the target protein variants; and
(g) identifying host cells that express the target protein variants.

15. The method of claim 14, wherein coding elements comprising variant sequences and coding elements comprising wild-type sequences are comprised by separate nucleic acid molecules

16. A nucleic acid molecule as defined in any one of claims 1 to 9.

17. A vector comprising the nucleic acid molecule of claim 16.

18. A cell comprising the nucleic acid molecule of claim 16 or the vector of claim 17.

19. A kit comprising one or more items of the group selected from a nucleic acid molecule according to claim 16, a vector according to claim 17, a cell according to claim 18, an RNA library or a (poly)peptide library according to claim 11 and, optionally, instructions for use.
